(19) Europäisches Patentamt
European Patent Office
Office européen des brevets

(11) Veröffentlichungsnummer: **0 100 033**
**B1**

(12) # EUROPÄISCHE PATENTSCHRIFT

(45) Veröffentlichungstag der Patentschrift: **14.01.87**

(21) Anmeldenummer: **83106938.0**

(22) Anmeldetag: **15.07.83**

(51) Int. Cl.⁴: **C 07 D 279/28**, C 07 D 279/26, A 61 K 31/54 // C07C93/06

(54) **Substituierte (10H-Phenothiazin-10-yl)propyl-1-piperazin-Derivate, Verfahren zu deren Herstellung sowie Arzneimittel enthaltend diese Verbindungen.**

(30) Priorität: **28.07.82 US 402704**

(43) Veröffentlichungstag der Anmeldung: **08.02.84 Patentblatt 84/6**

(45) Bekanntmachung des Hinweises auf die Patenterteilung: **14.01.87 Patentblatt 87/3**

(84) Benannte Vertragsstaaten: **AT BE CH DE FR GB IT LI LU NL SE**

(56) Entgegenhaltungen:
**DE-A-3 013 502**
**GB-A-853 803**
**US-A-3 081 229**

**Burger's Medicinal Chemistry, 4. Auflage, Teil III, S. 881-4**
**Medicinal Chemistry, 3. Auflage, Teil I, S. 75-6**

(73) Patentinhaber: **F. HOFFMANN- LA ROCHE & CO. Aktiengesellschaft, CH- 4002 Basel (CH)**

(72) Erfinder: **Davidson, Arnold B., 9 Knollwood Drive, North Caldwell, N.J. 07006 (US)**
Erfinder: **Guthrie, Robert William, 102 Alberta Drive, Saddle Brook, N.J. 07612 (US)**
Erfinder: **Kierstead, Richard Wightman, 30 Willowbrook Drive, North Caldwell, N.J. 07006 (US)**
Erfinder: **Ziering, Albert, 21 Oakley Terrace, Nutley, N.J. 07110 (US)**

(74) Vertreter: **Kellenberger, Marcus, Dr., Grenzacherstrasse 124 Postfach 3255, CH- 4002 Basel (CH)**

EP 0 100 033 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vörliegende Erfindung betrifft substituierte (1OH-Phenothiazin-10-yl)propyl-1-piperazine der allgemeinen Formel

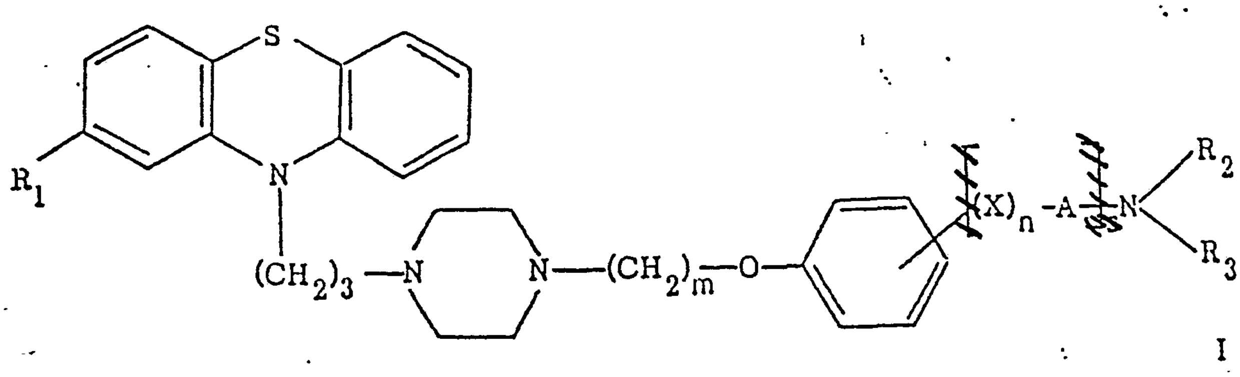

I

worin $R_1$ Wasserstoff, Halogen, $C_1$-$C_7$-Alkyl, $C_1$-$C_7$-Alkyl-thio, $C_1$-$C_7$-Alkoxy, Trihalomethyl oder die Gruppe -$SO_2NR_2R_3$, X Sauerstoff oder Schwefel, m eine Zahl von 2 bis 6, n die Zahl 0 oder 1, A $C_1$-$C_7$-Alkylen und, falls X Sauerstoff bedeutet, auch 2-Hydroxytrimethylen, und $R_2$ und $R_3$ unabhängig voneinander Wasserstoff oder $C_1$-$C_7$-Alkyl oder zusammen mit dem Stickstoffatom Morpholino oder Phthalimido bedeuten, die Enantiomeren der Verbindungen, worin A-Hydroxytrimethylen bedeutet, und pharmazeutisch verwendbare Säureadditionssalze davon.

Aus der U.S. Patentschrift 2.838.507 sind u.a. 1-, 2-, 3- oder 4-Halo-10-[γ-(N'-phenoxyalkyl-N-piperazino)propyl)phenothiazine bekannt, welche sich aufgrund ihrer pharmakologischen Eigenschaften insbesondere als Antiemetika, Ataraktika und Sedativa verwenden lassen.

Der in der vorliegenden Beschreibung verwendete Ausdruck "$C_1$-$C_7$-Alkyl" bedeutet geradkettige oder verzweigte gesättigte Kohlenwasserstoffreste mit 1 bis 7 Kohlenstoffatomen, wie Methyl, Aethyl, Propyl, Isopropyl, Butyl, tert.-Butyl, Neopentyl, Pentyl, Heptyl und dergleichen. Der Ausdruck "$C_1$-$C_7$-Alkoxy" bedeutet eine Alkyläthergruppe, in welcher die $C_1$-$C_7$-Alkylgruppe die obige Bedeutung besitzt, wie Methoxy, Aethoxy, Propoxy, Butoxy, Isobutoxy, Pentoxy und dergleichen. Der Ausdruck "$C_1$-$C_7$-Alkylthio" bedeutet eine Alkylthioäthergruppe, worin die $C_1$-$C_7$-Alkylgruppe die obige Bedeutung hat, wie Methylthio, Aethylthio, Propylthio, Butylthio, Isobutylthio, Pentylthio und dergleichen. Die Ausdrücke "Halogen" "Halo" und "HAL" bedeuten die Halogene Brom, Chlor, Fluor und Jod. Der Ausdruck "$C_1$-$C_7$-Alkylen" bedeutet einen divalenten geradkettigen oder verzweigten gesättigten Kohlenwasserstoffrest mit 1 bis 7 Kohlenstoffatomen, wie Methylen, Aethylen, Trimethylen, Tetramethylen, 1,1-Dimethyl-äthylen, Pentamethylen und dergleichen.

Eine bevorzugte Cruppe von Verbindungen der Formel sind solche, worin X Sauerstoff und n die Zahl 1 bedeuten.

Eine noch mehr bevorzugte Gruppe von Verbindungen der Formel 1 sind solche, worin X Sauerstoff, $R_2$ und $R_3$ unabhängig voneinander Wasserstoff oder $C_1$-$C_7$-Alkyl und n die Zahl 1 bedeuten.

Eine besonders bevorzugte Gruppe von Verbindungen der Formel I sind solche, worin $R_1$ Halogen oder Trifluormethyl, X Sauerstoff, m die Zahl 2, n die Zahl 1 und $R_2$ und $R_3$ unabhängig voneinander Wasserstoff oder $C_1$-$C_7$- Alkyl bedeuten.

Eine ganz besonders bevorzugte Verbindung der Formel I ist.

(S)-1-(1-Methyläthylamino)-3-[4-[2-]4-[3-(2-chlor-1H-phenothiazin-10-yl)propyl]piperazin-1-yl]äthoxy]-phenoxy]-2-propanol

Weitere bevorzugte Verbindungen der Formel I sind:

(R)-1-(1-Methyläthylamino)-3-[4-[2-[4-[3-(2-chlor-10H-phenothiazin-10-yl)propyl]piperazin-1-yl]äthoxy]-phenoxy%]-2-propanol;

(S)-1-(1-Methyläthylamino)-3-[4-[2-[4-[3-(2-trifluor-methyl-10H-phenothiazin-10-yl)propyl]piperazin-1-yl]-athoxy)phenoxy]-2-propanol;

1-[3-(2-Chlor-10H-phenothiazin-10-yl)propyl]-4-[2-[4-[2-(1-methyläthyl)aminoäthoxy]phenoxy]äthyl]piperazin;

1-[3-(2-Chlor-10H-phenothiazin-10-yl)propyl]-4-[2-[-[3-(1-methyläthyl)aminopropoxy]phenoxy]äthyl]piperazin;

1-[3-(2-Chlor-10H-phenothiazin-10-yl)propyl]-4-[2-[4-[4-(1-methyläthyl)aminobutoxy]phenoxy]äthyl]piperazin;

1-[3-(2-Chlor-10H-phenothiazin-10-yl)propyl]-4-[2-[4-[5-(1-methyläthyl)aminopentoxy]phenoxy]äthyl]piperazin;

1-[3-(2-Methylthio-10H-phenothiazin-10-yl)propyl]-4-[2-[4-[5-(1-methyläthyl)aminopentoxy]phenoxy]äthyl]piperazin;

1-[3-(2-Chlor-10H-phenothiazin-10-yl)-4-[2-[4-[6-(1-methyläthyl)aminöhexyloxy]phenoxy]äthyl]piperazin;

(S)-1-(1-Methyläthylamino)-3-[4-[2-[4-[3-(2-N,N-dimethylsulfamoyl-10H-phenothiazin-10-yl)propyl]piperazin 1-yl]äthoxy]phenoxy]-2-propanol;
(S)-1-(1-Methyläthylamino)-3-[4-[2-[4-[3-(2-methoxy-10H-phenothiazin-10-yl)propyl]piperazin-1-yl]äthoxy]-phenoxy]-2-propanol;
(S)-1-(1-Methyläthylamino)-3-[4-[2-[4-[3-(2-methyl-thio-10H-phenothiazin-10-yl)propyl]piperazin-1-yl]äthoxy]-phenoxy]-2-propanol;
(S)-1-(1-Methyläthylamino)-3-[4-[2-[4-[3-(2-methyl-10H-phenothiazin-10-yl)propyl]piperazin-1-yl]äthoxy]phenoxy]-2-propanol;
1-[3-(2-Chlor-10H-phenothiazin-10-yl)propyl]-4-[2-[4-[2-(1-methyläthyl)aminoäthyl]phenoxy]äthyl]piperazin;
1-[3-(2-Chlor-10H-phenothiazin-10-yl)propyl]-4-[2-[4-[3-(1-methyläthyl)aminopropyl]phenoxy]äthyl]piperazin;
1-[3-(2-Chlor-10H-phenothiazin-10-yl)propyl]-4-[2-[4-[4-(1-methyläthyl)aminobutyl]phenoxy]äthyl]piperazin;

Die Verbindungen der Formel I und deren pharmazeutisch verwendbare Säureadditionssalze können erfindungsgemäss dadurch hergestellt werden, dass man

a) eine Verbindung der allgemeinen Formel

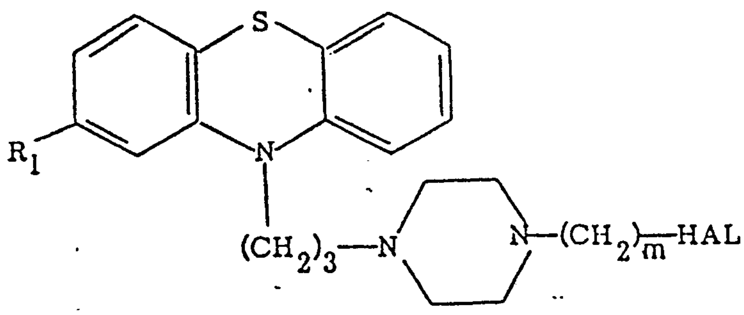

II

worin $R_1$ und m die obige Bedeutung besitzen, mit einer Verbindung der allgemeinen Formel

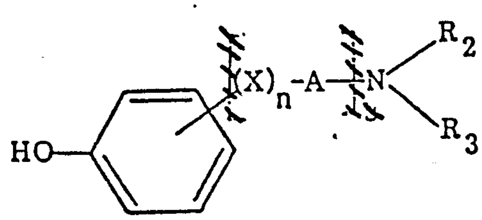

III

worin $R_2$, $R_3$, A, X und n die obige Bedeutung besitzen, umsetzt und

b) erwunschtenfalls eine erhaltene Verbindung, worin $R_2$ und $R_3$ zusammen mit dem Stickstoffatom Phthalimido bedeuten, durch Hydrazinolyse in die entsprechende Verbindung überführt worin $R_2$ und $R_3$ Wasserstoff bedeuten, und/oder

c) erwünschtenfalls ein erhaltenes Racemat einer Verbindung, worin A 2-Hydroxytrimethylen bedeutet, in die optisch aktiven Antipoden auftrennt und

d) erwünschtenfalls eine erhaltene Verbindung oder ein nicht pharmazeutisch verwendbares Säureadditionssalz in ein pharmazeutisch verwendbares Säureadditionssalz überführt.

Gemäss Verfahrensvariante a) wird eine Verbindung der Formel II mit einer Verbindung der Formel III in einer unter üblichen Reaktionsbedingungen zu einer Verbindung der Formel I umgesetzt. Ueblicherweise wird die Reaktion in Gegenwart einer Base wie Natriumhydroxid, Kaliumhydroxid, Natriumcarbonat, Kaliumcarbonat und dergleichen, und eines Lösungsmittels, wie wassriges Dimethylsulfoxid, wässriges Dimethylformamid und dergleichen, durchgeführt. Vorzugsweise erfolgt die Umsetzung bei einer Temperatur im Bereich von etwa 20° C bis etwa 100° C, besonders bevorzugt bei etwa 50° C.

Die erhaltene Verbindung der Formel I kann nach üblichen Extraktionsmethoden und unter Verwendung üblicher Lösungsmittel isoliert werden. Zweckmässigerweise wird die erhaltene Verbindung der Formel I nach üblichen Methoden wie weiter unten beschrieben in ein Säureadditionssalz übergeführt. Das gebildete Salz kann durch Kristallisation oder ähnlichen Verfahren isoliert werden.

Gemäss Verfahrensvariante b) wird eine Verbindung der Formel I, worin $R_2$ und $R_3$ zusammen mit dem

Stickstoffatom Phthalimido bedeuten, in einem inerten Lösungsmittel, wie Aethanol oder wässriges Aethanol, bei einer Temperatur zwischen etwa Raumtemperatur und etwa 100°C, vorzugsweise bei der Rückflusstemperatur, mit Hydrazin unter Bildung des entsprechenden primären Amins umgesetzt, d.h. einer Verbindung der Formel I, worin $R_2$ und $R_3$ Wasserstoff bedeuten.

Die Verbindungen der Formel I bilden Säureadditionssalze mit anorganischen oder organischen Säuren. So bilden sie pharmazeutisch verwendbare Säureadditionssalze mit pharmazeutisch verwendbaren organischen und anorganischen Säuren, z.B. mit Hydrohalogensäuren, wie Chlorwasserstoffsäure, Bromwasserstoffsäure, Jodwasserstoffsäure, mit anderen Mineralsäuren, wie Schwefelsäure, Salpetersaure, Phosphorsäure, oder dergleichen, mit Alkyl- und mono-Arylsulfonsäuren, wie Aethansulfonsäure, Toluolsulfonsäure, Benzolsulfonsäure oder dergleichen, mit anderen organischen Säuren, wie Essigsäure, Weinsäure, Maleinsäure, Zitronensäure, Benzoesäure, Salicylsäure, Ascorbinsäure und dergleichen. Pharmazeutisch nicht verwendbare Säureadditionssalze von Verbindungen der Formel I können durch übliches Umsalzen in pharmazeutisch verwendbare Säureadditionssalze übergeführt werden, wobei das pharmazeutisch nicht verwendbare Anion durch ein pharmazeutisch verwendbares Anion ersetzt wird. Alternativ dazu kann man ein pharmazeutisch nicht verwendbares Säureadditionssalz auch neutralisieren und danach die so erhaltene freie Base mit einem Reagens umsetzen, welches ein pharmazeutisch verwendbares Säureadditionssalz liefert.

Die Verbindungen der Formel I, worin A 2-Hydroxytri-methylen bedeutet, und deren pharmazeutisch verwendbare Säureadditionssalze besitzen ein asymmetrisches Kohlenstoffatom. Diese Verbindungen werden gewöhnlicherweise als racemische Mischungen erhalten. Die Auftrennung der einzelnen Racemate in die optisch aktiven Isomeren d.h. Enantiomeren, erfolgt nach üblichen Methoden. Alternativ dazu können auch optisch aktive Isomere hergestellt werden, indem man von optisch aktiven Ausgangsstoffen ausgeht. Einige racemische Mischungen können als Eutektika ausgefällt werden und können danach getrennt werden. Die chemische Auftrennung ist jedoch bevorzugt. Gemäss dieser chemischen Methode wird das erwünschte Enantiomere gebildet, indem man das racemische Gemisch mit einem optisch aktiven Trennungsmittel, beispielsweise einer optisch aktiven Säure, wie (+)-Weinsäure, (+)-Dibenzoyl-D-weinsäure, (+)-d-10-Camphersulfonsäure, (-)-3-Pinancarbonsäure und dergleichen unter Bildung von einem enantiomeren Salz umsetzt. Die gebildeten Enantiomeren werden durch fraktionierte Kristallisation aufgetrennt und können danach in die entsprechenden optisch aktiven Basen überführt werden. Demnach betrifft die Erfindung sowohl die optisch aktiven Isomeren der Verbindungen der Formel I, worin A 2-Hydroxy-trimethylen bedeutet, als auch deren Racemate.

Die Ausgangsstoffe der Formel II sind bekannt oder können nach bekannten Methoden hergestellt werden.

Beispiele von Verbindungen der Formel II sind:

1-[3-(2-Chlor-10H-phenothiazin-10-yl)propyl]-4-(2-chloräthyl)piperazin;

1-[3-(2-Chlor-10H-phenothiazin-10-yl)propyl]-4-(3-chlorpropyl)piperazin;

1-[3-(2-Trifluormethyl-10H-phenothiazin-10-yl)-propyl]-4-(2-chloräthyl)piperazin;

1-[3-(2-Chlor-10H-phenothiazin-10-yl)propyl]-4-(4-chlorbutyl)piperazin;

1-[3-(2-Methylthio-10H-phenothiazin-10-yl)propyl]-4-(2-chloräthyl)piperazin; und dergleichen.

Die Ausgangsstoffe der Formel III sind ebenfalls bekannt oder können nach bekannten Methoden hergestellt werden.

Beispiele von Verbindungen der Formel III sind:

a) 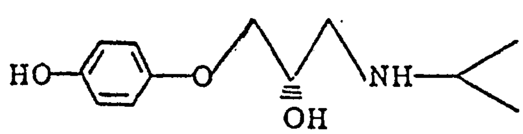

b) 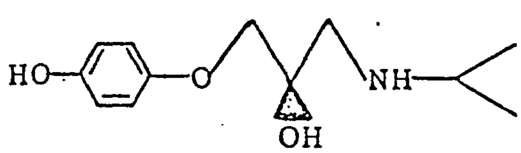

c) 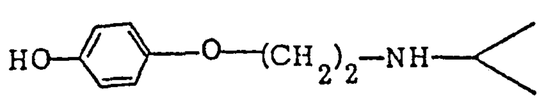

d) 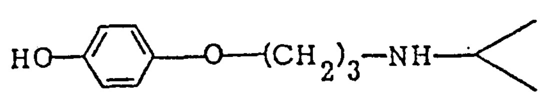

e) 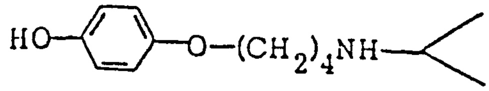

f) 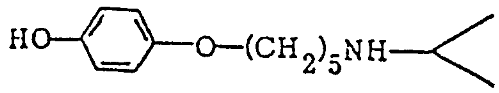

g) 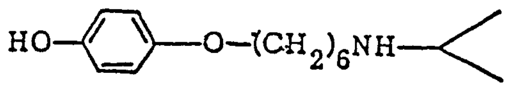

h) 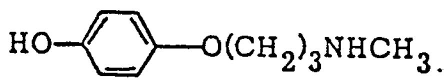

i) 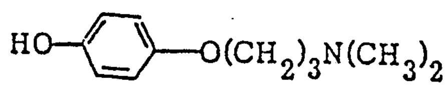

j) 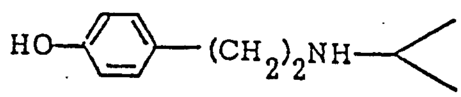

k) 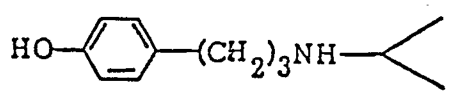

l) 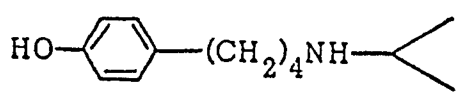

m) 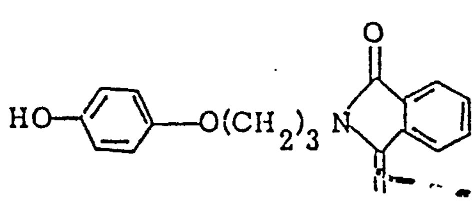

Die Verbindungen der Formel I und deren pharmazeutisch verwendbare Säureadditionssalze zeigen langanhaltende neuroleptische Aktivität. Folglich sind die Verbindungen der Formel I als langwirksame antipsychotische Mittel brauchbar, z.B. bei der Behandlung von Schizophrenie. Die Aktivität der Verbindungen der Formel I, welche diese als orale antipsychotische Mittel brauchbar macht, kann nach bekannten Arbeitsweisen an Warmblütlern demonstriert werden.

Nach einer Arbeitsweise werden z.B. trainierte Ratten in Versuchskammerngebracht, die mit einem Reaktionshebel, einem Stahlgitterbcden zum Verabreichen eines Elektroschocks und einem Lautsprecher für akustische Stimulation ausgestattet sind. Jeder Versuch besteht in einem Warnton von 15 Sekunden (bedingter Stimulus), der für weitere 15 Sekunden seine Fortsetzung findet und dabei von einem Elektroschock begleitet wird (unbedingter Stimulus; 1,0 mA, 350 V-Wechselspannung). Die Ratten können einen Versuch jederzeit durch Niederdrücken des Reaktionshebels beenden. Eine Reaktion während des ersten 15 Sekunden dauernden Warntons beendet den Versuch, bevor ein Schock verabreicht wird, und wird als Vermeidungsreaktion angesehen, während eine Reaktion, die während der Schockausteilung erfolgt, eine Fluchtreaktion ist. Versuche werden alle 2 Minuten während einer 1-stündigen Testdauer vorgenommen (30 Versuche pro Testdauer).

Trainierte Ratten zeigen eine verlässliche Grundlinie des Vermeidungsverhaltens (0-3 ausbleibende Vermeidungsreaktionen pro Testdauer). Mindestens 3-4 Ratten erhalten die Testverbindungen in jeder Dosierungsmenge über einen Dosierungsbereich verabreicht. Vor Verabreichung der Testverbindungen erhalten die Ratten zur Kontrolle das Trägermittel allein. Nach einmaliger Verabreichung der Testverbindung wird jede Ratte täglich oder wöchentlich getestet, bis das Vermeidungsverhalten die Grundlinie vor Verabreichung der Testsubstanz wie

Jede Versuchsdauer ist in drei aufeinanderfolgende 20-minütige Abschnitte (10 Versuche) unterteilt. Die Anzahl der Reaktionen wird über alle Individuen bei gegebener Dosis innerhalb jedes Abschnitts summiert. Die Anzahl der Versuche, bei denen die Ratten keine Vermeidungsreaktion (Vermeidungsblockierung, VB) oder keine Fluchtreaktion (Fluchtblockierung, FB) zeigten, wird für den Abschnitt bestimmt, der bei jeder Dosis den maximalen Effekt der innerhalb des Abschnitts insgesamt vorgenommenen Versuche ergibt. Die für 50% Vermeidungsblockierung (VB 50) berechnete Dosis wird aus der Dosis-Wirkung-Regressionslinie erhalten, aufgestellt nach der Methode der kleinsten Quadrate. Die geringste Dosis, die 20% Blockierung der Fluchtreaktion (FB 20) bewirkt, wird aus einem Dosiswirkungs-Diagramm abgelesen. Um diese Werte zu erhalten, wird die Wirkung in % gegen den Logarithmus der Dosis aufgetragen.

Antipsychotische Mittel können von anderen Wirkstoffarten, die das Verhalten von Ratten bei dieser Arbeitsweise beeinträchtigen, durch die grössere Trennung zwischen Dosen, die Vermeidungsreaktion blockieren, und Dosen, die Fluchtreaktion blockieren, unterschieden werden. Die klinische Potenz antipsychotischer Wirkstoffe mit bekannten therapeutischen Anwendungen und Eigenschaften hängt wesentlich und stark mit ihrer Potenz in diesem Versuchsprozedere zusammen. Folglich können die Verbindungen der Formel I therapeutisch in Dosierungen eingesetzt werden, die mit ihrer Potenz im Testverfahren in Uebereinstimmung stehen.

Wenn (S)-1-(1-Methyläthylamino)-3-[4-[2-[4-[3-(2-chlor-10H-phenothiazin-10-yl)propyl]piperazin-1-yl]äthoxy]-phenoxy]-2-propanol-trihydrochlorid (Verbindung A), welches nach einmaliger Verabreichung eine $LD_{50}$ von z.B. 45 mg/kg p.o. nach 7 Tagen und 77,5 mg/kg i.p. nach 24 Stunden bei Mäusen zeigt, als Testsubstanz verwendet wird, wird eine maximale neuroleptische Aktivität bei einer $VB_{50}$ von 13,5 mg/kg p.o. 7-10 Tage nach Vereinbarung beobachtet.

In diesem diskontinuierlichen Vermeidungsverfahren, von welchem mit grosser Wahrscheinlichkeit auf die antipsychotische Wirkung geschlossen werden kann, ist die Vermeidungsblockierung der Verbindung A sehr spezifisch, mit nur geringer oder gar keiner Fluchtblockierung. Die neroleptische Wirkung hielt für einige Wochen an.

Die relative antipsychotische Wirkung der Verbindungen der Formel I sowie deren Wirkungsdauer in diesem diskontinuierlichen Vermeidungstest sind in Tabelle 1 zusammengestellt.

Die verlängerte Wirkungsdauer der Verbindung A kann auch in anderen für Neuroleptika spezifischen Testanordnungen gezeigt werden. Diese Resultate sind in Tabelle 2 zusammengefasst. Die Wirkung von Verbindung A in einigen Versuchsanordnungen, welche geeignet sind, neuroleptische Wirkung zu zeigen, sind in Tabelle 3 zusammengefasst.

**Tabelle 1**

**Diskontinuierliche Vermidung - Ratten**

| Verbindung | Dosis mg/kg p.o. | % Vermeidungsblockierung 60 Min. nach Verabreichung | 24 Std. nach Verabreichung | Zeitpunkt der Hauptwirkung (VB)* | Dauer einer signifikanten Wirkung (VB)* |
|---|---|---|---|---|---|
| (S)-1-(1-Methyläthylamino)-3-[4-[2-[4-[3-(2-chlor-10H-phenothiazin-10-yl)propyl]piperazin-1-yl]äthoxy]-phenoxy]-2-propanol-trihydrochlorid | 40 | 40% | 77% | 2-10 Tage (90%) | 8 Wochen (30%) |
| 1-[3-(2-Chlor-10H-phenothiazin-10-yl)propyl]-4-[2-[4-[2-(1-methyläthyl)-aminoäthoxy]phenoxy]äthyl]piperazin-trihydrochlorid | 40 | 10% | 100% | 1 Tag (100%) | 8 Wochen (37%) |
| 3-[[4-[2-[4-[3-(2-Chlor-10H-phenothiazin-10-yl)propyl]-1-piperazinyl]äthoxy]phenyloxy]-N,N-dimethylpropanamin-trihydrochlorid | 40 | 50% | 100% | 1 Tag (100%) | >6 Wochen (25%) |
| (S)-1-(1-Methyläthylamino)-3-[4-[2-[4-[3-(2-trifluormethyl)-10H-phenothiazin-10-yl)propyl]piperazin-1-yl]-äthoxy]phenoxy]-2-propanol-trihydrochlorid | 25 | 33% | 57% | 3-10 Tage (77-87%) | 6 1/2 Wochen (37%) |

*(VB) = Vermeidungsblockierung zu gegebener Zeit

**Tabelle 1** (Fortsetzung)

**Diskontinuierliche Vermeidung - Ratten**

| Verbindung | Dosis mg/kg p.o. | % Vermeidungsblockierung 60 Min. nach Verabreichung | % Vermeidungsblockierung 24 Std. nach Verabreichung | Zeitpunkt der Hauptwirkung (VB)* | Dauer einer signifikanten Wirkung (VB)* |
|---|---|---|---|---|---|
| 3-[[4-[2-[4-[3-(2-Chlor-10H-phenothiazin-10-yl)propyl]-1-piperazinyl]äthoxy]phenyl]oxy]propanamin-trihydrochlorid | 40 | 40% | 73% | 3 Tage (90%) | 5 1/2 Wochen (27%) |
| 1-[3-(2-Chlor-10H-phenothiazin-10-yl)propyl]-4-[2-[4-[4-(1-methyläthyl)aminobutyl]phenoxy]äthyl]-piperazin | 40 | 17% | 100% | 1 Tag (100%) | 5 1/2 Wochen (55%) |
| 1-[3-(2-Chlor-10H-phenothiazin-10-yl)propyl]-4-[2-[4-[2-(1-methyläthyl)-aminoäthyl]phenoxy]äthyl]piperazin-trimaleat | 40 | 10% | 100% | 1 Tag (100%) | 5 Wochen (80%) |
| 1-[3-(2-Chlor-10H-phenothiazin-10-yl)propyl]-4-[2-[4-[3-(1-methyläthyl)-aminopropyl]phenoxy]äthyl]piperazin-trihydrochlorid | 40 | 45% | 100% | 1 Tag (100%) | 5 Wochen (55%) |
| 3-[[4-[2-[4-[3-(2-Chlor-10H-phenothiazin-10-yl)propyl]-1-piperazinyl]äthoxy]phenyl]oxy]-N-methyl-propanamin-trihydrochlorid | 40 | 65% | 100% | 1 Tag (100%) | 5 Wochen (25%) |

**Tabelle 1** (Fortsetzung)

**Diskontinuierliche Vermeidung - Ratten**

| Verbindung | Dosis mg/kg p.o. | % Vermeidungsblockierung 60 Min. nach Verabreichung | 24 Std. nach Verabreichung | Zeitpunkt der Hauptwirkung (VB)* | Dauer einer signifikanten Wirkung (VB)* |
|---|---|---|---|---|---|
| 1-[3-(2-Chlor-10H-phenothiazin-10-yl)propyl]-4-[2-[4-[3-(1-methyläthyl)-aminopropoxy]phenoxy]äthyl]piperazin —trihydrochlorid | 40 | 10% | 97% | 1 Tag (97%) | 5 Wochen (50%) |
| 1-[3-(2-Chlor-10H-phenothiazin-10-yl)-propyl]-4-[2-[4-[4-(1-methyläthyl)-aminobutoxy]phenoxy]äthyl]piperazin-trihydrochlorid | 40 | 20% | 33% | 3-10 Tage (97-100%) | 5 Wochen (30%) |
| (R)-1-(1-Methyläthylamino-3-[4-[2-[4-[3-(2-chlor-10H-phenothiazin-10-yl)-propyl]piperazin-1-yl]äthoxy]-phenoxy]-2-propanol-trihydrochlorid | 40 | 20% | 27% | 10 Tage (37%) | 3 Wochen (27%) |
| 1-[3-(2-Chlor-10H-phenothiazin-10-yl)-propyl]-4-[2-[4-[5-(1-methyläthyl)-aminopentoxy]phenoxy]äthyl]piperazin-trimaleat | 40 | 10% | 97% | 1 Tag (97%) | 2 Wochen (23%) |
| 1-[3-(2-Chlor-10H-phenothiazin-10-yl)-4-[2-[4-[6-(1-methyläthyl)amino-hexyloxy]phenoxy]äthyl]piperazin-trihydrochlorid | 40 | 23% | 40% | 1 Tag (40%) | 1 Woche (30%) |
| 1-[3-(2-Methylthio-10H-phenothiazin-10-yl)propyl]-4-[2-[4-[5-(1-methyl-äthyl)aminopentoxy]phenoxy]äthyl]-piperazin-trimaleat | 40 | 60% | 45% | 60 Min. (60%) | 3 Tage (35%) |

**Tabelle 2**

**Wirkung der Verbindung A* nach einmaliger Verabreichung**

| Test | 1-4 Std. | 2-4 Std. | 1 Woche | 2 Wochen | 3 Wochen | 4 Wochen | 5 Wochen | 6 Wochen | 7 Wochen | 8 Wochen |
|---|---|---|---|---|---|---|---|---|---|---|
| Diskontinuierliche Vermeidung % Blockierung (Ratten, 40 mg/kg p.o.) | 40% | 78% | 84% | 80% | 84% | 82% | 56% | 50% | 31% | 30% |
| Stabsprung-Schockvermeidung % Blockierung (Ratten, 40 mg/kg p.o.) | 30% | - | 50% | 44% | 11% | - | - | - | - | - |
| Kontinuierliche Vermeidung % Abnahme der Reaktion (Ratten, 40 mg/kg p.o.) | 0% | 59% | 97% | 96% | 93% | 84% | 82% | 55% | 48% | 32% |
| Kontinuierliche Vermeidung % Abnahme der Reaktion (Totenkopfaffen, 5 mg/kg p.o.) | 0% | 15% | 35% | 37% | 24% | - | - | - | - | - |
| Apomorphin-induzierte Emesis % Antagonismus (Hunde, mg/kg s.c.) | 93% | 100% | 100% | 100% | 100% | 100% | 100% | 100% | 56% | 40% |
| Serum-Prolactin-Spiegel % Zunahme (Ratten, 15 mg/kg p.o.) | 215% | 97% | 525% | - | 251% | 56% | - | - | - | - |
| Amphetamin-induzierte Rotation % Antagonismus (S.Nigra-verletzte Ratten, 40 mg/kg p.o.) | 14% | - | 97% | 95% | 87% | 87% | 62% | 28% | 4% | - |

* Verbindung A = (S)-1-(1-Methyläthylamino)-3-[4-[2-[4-[3-(2-chlor-10H-phenothiazin-10-yl)-propyl]piperazin-1-yl]äthoxy]phenoxy]-2-propanol-trihydrochlorid

**Tabelle 3**

| Test | Spezies | Verbindung A (7 Tage nach Verabreichung) | Haloperidol (1 Stunde nach Verabreichung) | Chlorpromazin (1 Stunde nach Verabreichung) |
|---|---|---|---|---|
| Diskontinuierliche Vermeidung $VB_{50}$ (mg/kg, p.o.) | Ratte | 19 | 0,35 | 5 |
| Quotient $FB_{20}/VB_{50}$ | | >2,1 | 7,8 | 9,6 |
| Kontinuierliche Vermeidung MED Schocks (mg/kg p.o.) | Totenkopfaffe | <5 | 0,28 | 2 |
| Rotation-Antagonismus $ED_{50}$ (mg/kg p.o.) | Ratte | <15 | 0,35 | 20,5 |
| Einschränkung der motorischen Aktivität $DD_{50}$ (mg/kg p.o.) | Ratte | 19,6 | 0,4 | 4,6 |
| Anti-Apomorphin-Emesis $ED_{50}$ (mg/kg s.c.) | Hund | <5 | 0,02 | 1,4 |
| Inhibition der 3H-Spiroperidol-Bindung im Caudatum, $IC_{50}$ (nM) | Ratte (in vitro) | 1,8 | 6,2 | 11 |
| Dopamin-Umsatz im Gehirn;<br>im Caudatum;<br>im limbischen System und Rest | Ratte | 40 mg/kg p.o.<br>signif. ↑<br>nicht signif. | 1 mg/kg i.p.<br>signif. ↑<br>signif. ↑ | 10 mg/kg i.p.<br>signif. ↑<br>signif. ↑ |
| Dopamin-Adenylat-Cyclase-Inhibition im limbischen System, $IC_{50}$ (nM) | Ratte (in vitro) | 500 | 250 | 150 |
| Prolactin-Ausschüttung $ID_{50}$ (nM) | Ratte (in vitro) | 15 | 27 | 130 |

Die Verbindungen der Formel I und ihre pharmazeutisch verwendbaren Säureadditionssalze besitzen antipsychotische Wirkungen, die denen von Haloperidol und Chlorpromazin, welche durch ihre therapeutische Verwendung und Eigenschaften bekannt sind, qualitativ ähneln, mit Ausnahme der Wirkungsdauer. (S)-1-(1-Methyläthylamino)-3-[4-[2-[4-[3-(2-chlor-10H-phenothiazin-10-yl)propyl]piperazin-1-yl]äthoxy]phenoxy] 2-propanol-trihydrochlorid bewirkte zwar bei den höchsten im Ames-Test zur Bestimmung der Mutagenität geprüften Konzentrationen eine Zunahme an zurückmutierten Kolonien, doch können kein schlüssigen Aussagen über mutagene Effekte in Warmblütlern gemacht werden, da keine Daten vorliegen, welche in einem Testsystem für Säugetiere bestimmt worden sind.

Die Verbindungen der Formel I und ihre pharmazeutisch verwendbaren Säureadditionssalze können in Form herkömmlicher pharmazeutischer Zubereitungen verwendet werden. Geeignete orale Dosierungseinheiten enthalten beispielsweise oder liegen im Bereich von 1-500 mg. Geeignete orale Dosierungen bei Warmblütlern sind oder liegen im Bereich von ungefähr 0,01 mg/kg bis 50 mg/kg pro Tag, verabreicht in Intervallen von 3 Tagen bis 8 Wochen. Für jeden bestimmten Warmblütler kann jedoch die spezifische Dosierung schwanken und sollte nach den individuellen Bedürfnissen und dem fachkundigen Urteil der die Verabreichung einer Verbindung der Formel I oder eines pharmazeutisch verwendbaren Säureadditionssalzes davon vornehmenden oder überwachenden Person eingestellt werden. Im weiteren schwankt die Häufigkeit, mit der jede solche Dosierungsform verabreicht wird, in Abhängigkeit von der Menge an vorhandenem aktivem Wirkstoff und den Erfordernissen der pharmakologischen Situation.

Für die offenbarte Verwendung werden die Verbindungen der Formel I und deren pharmazeutisch verwendbare Säureadditionssalze unter Einsatz herkömmlicher inerter pharmazeutischer Hilfsstoffe zu Dosierungsformen verarbeitet, welche sich für die orale oder parenterale Verabreichung eignen. Solche Dosierungsformen umfassen Tabletten, Suspensionen, Lösungen und dergleichen. Ferner können die Verbindungen der Formel I in geeignete harte oder weiche Kapseln eingearbeitet oder in Form geeigneter harter oder weicher Kapseln verabreicht werden. Die Art der inerten Hilfsstoffe, die zum Verarbeiten der Verbindungen der Formel I und ihrer pharmazeutisch verwendbaren Säureadditionssalze zu oralen oder parenteralen Dosierungsformen verwendet werden, liegen für den Fachmann sofort auf der Hand. Diese entweder anorganischen oder organischen Hilfsstoffe umfassen z.B. Wasser, Gelatine, Lactose, Stärke, Magnesiumstearat, Talk, pflanzliche Oele, Gummi arabicum, Polyalkylenglykole und dergleichen. Ferner können erwünschtenfalls Konservierungsmittel, Stabilisierungsmittel, Benetzungsmittel, Emulgatoren, Salze zur Veränderung des osmotischen Druckes, Puffer und dergleichen in solche Darreichungsformen eingearbeitet werden.

Die folgenden Beispiele veranschaulichen die Erfindung noch weiter. Alle Temperaturen sind in Celsiusgraden angegeben.

**Beispiel 1**

A) 4-[3-(2°Chlorphenothiazin-10-yl)propyl]-1-piperazin-äthanol (22,5 g) werden zu 188 ml trockenem Methylenchlorid welches 7,8 ml Triäthylamin enthält, gegeben. Die gerührte Lösung wird auf -10° gekühlt und bei dieser Temperatur gehalten, während man tropfenweise eine Lösung von 5,1 ml Mesylchlorid in 50 ml trockenem Methylenchlorid zugibt. Das Eisbad wird danach entfernt, und das Reaktionsgemisch wird über Nacht bei Raumtemperatur gerührt. Die Lösung wird mit 25 ml einer 5%igen Natriumbicarbonatlösung gewaschen und danach über wasserfreiem Kaliumcarbonat getrocknet. Das Lösungsmittel wird unter vermindertem Druck entfernt (Badtemperatur 25°), wobei man 24,2 g 1-[3-(2-Chlor-10H-phenothiazin-10-yl)propyl]-4-(2-chloräthyl)piperazin als Oel erhält.

Diese Verbindung wird in den nachfolgenden Reaktionen als solche verwendet oder zunächst in das entsprechende Dihydrochlorid übergeführt und als Salz verwendet. So wird in einem separaten Experiment die Base 1-[3-(2-Chlor-10H-phenothiazin-10-yl)propyl]-4-(2-chloräthyl)piperazin (75 g) in 700 ml Methanol mit überschüssiger methanolischer Salzsäure (75 ml; ~ 3,5N) versetzt, wobei das Salz unmittelbar aus der Lösung auszukristallisieren beginnt. Nach 1-stündigem Rühren bei Raumtemperatur wird das Gemisch auf 0° abgekühlt, und die Kristalle werden abfiltriert, mit kaltem Methanol gewaschen und im Vakuum bis zur Gewichtskonstanz getrocknet, wobei man 69 g 1-[3-(2-Chlor-10H-phenothiazin-10-yl)propyl]-4-(2-chloräthyl)piperazindihydrochlorid vom Schmelzpunkt 147-149° erhält.

Analyse für $C_{21}H_{25}Cl_2N_3S.2HCl$:
Berechnet: C, 50,92, H, 5,49, N, 8,48, Cl, 28,63, S, 6,47
Gefunden: C, 51,11, H, 5,48, N, 8,69, Cl, 28,75, S, 6,77.

B.1) 263 g Bleitetraacetat werden in 1500 ml trockenem Benzol unter Argon suspendiert. Zur stark gerührten Mischung werden 140 g (2R,3R,4R,5R)-Mannitol-1,2,5,5-diacetonid über einen Zeitraum von 15 Minuten in 5-10 g-Portionen gegeben, und danach werden noch solange Portionen von 1 g Acetonid zugegeben, bis der Nachweis auf Oxidantien mittels Kaliumjodid/Stärke-Papier ein negatives Resultat liefert. So werden insgesamt 150 g (140 g + 10 + 1 g) an Acetonid eingesetzt. Das Reaktionsgemisch wird durch Celit filtriert, und der Filterkuchen wird zweimal mit 100 ml trockenem Benzol gewaschen. Zur Neutralisation der bei der Oxidation gebildeten Essigsäure wird das Filtrat während 30 Minuten mit 300 g wasserfreiem Kaliumcarbonat gerührt. Nach einer zweiten Filtrierung durch Celit wird die so erhaltene Lösung von D-Glyceraldehyd-acetonid

mit 450 ml Isopropylamin versetzt und über 15 g 10% Palladium auf Kohle bei einem Druck von einer Atmosphäre und bei einer Temperatur von 23° hydriert. Nach Aufnahme von 26,4 1 Wasserstoff stoppt die Reaktion im wesentlichen. Der Katalysator wird abfiltriert, und das Filtrat wird eingedampft, wobei man 188 g 2(S)-3-(1-Methyläthyl)amino-1,2-propandiol-acetonid erhält.

855 mg 2(S)-3-(1-Methyläthyl)amino-1,2-propandiol-acetonid in 20 ml Aether werden auf 0° gekühlt und mit 4 mMol Chlorwasserstoff in Aether versetzt. Der erhaltene Niederschlag wird abfiltriert und mit Aether gewaschen, wobei man 500 mg des Aminhydrochloridsalzes vom Schmelzpunkt 135-135° erhält; $[\alpha]_D^{25}$ -40,5° (c = 1,0% in Wasser).

Analyse für $C_9H_{18}NO_2.HCl$:
Berechnet: C, 51,55, H, 9,61, N, 6,68, C1, 16,91
Gefunden: C, 51,96, H, 9,89, N, 7,00, Cl, 16,62.

B.2) 90 ml Mesylchlorid werden unter Rühren zu einer vorgängig auf -10° gekühlten Lösung von (2S)-3-Isopropylamino-1,2-propandiol-acetonid (188 g; 1,087 Mol) und Triäthylamin (228 ml; 1,53 Mol) in trockenem Tetrahydrofuran so zugegeben, dass die Reaktionstemperatur 5° nicht übersteigt. Das Gemisch wird danach während 30 Minuten bei 10-15° gerührt, worauf es mit 1,5 1 einer gesättigten Kochsalzlösung verdünnt wird. Die beiden Phasen werden getrennt, und die wässrige Phase wird dreimal mit 500 ml Aether extrahiert. Die organischen Phasen ihrerseits werden mit zweimal 500 ml gesättigter Kochsalzlösung gewaschen und danach kombiniert, über Natriumsulfat getrocknet und eingedampft, wobei man 264 g (2S)-3-[N-Mesyl-(1-methyläthyl)amino]-1,2-propandiol-acetonid als Oel erhält.

Eine kleine Probe wird dreimal aus Hexan kristallisiert, wobei man analytisch reines Material vom Schmelzpunkt 33-34° erhalt; $[\alpha]_D^{25}$ -14,76° (c = 1% in Chloroform).

Analyse für $C_{10}H_{21}NO_4S$:
Berechnet: C, 47,79, H, 8,42, N, 5,57, S, 12,76
Gefunden: C, 47,87, H, 8,66, N, 5,72, S, 12,89.

B.3) 200 ml von mit Wasser und Methanol vorgewaschenem Dowex ®50W-8X Ionenaustauscherharz (H+-Form) werden zu einer Lösung von 264 g rohem (2S)-3-[N-Mesyl-(1-methyl-äthyl)amino]-1,2-propandiol-acetonid in 1 l Methanol und 325 ml Wasser gegeben. Das Gemisch wird während 90 Minuten unter Rückfluss gerührt. Die abgekühlte Mischung wird filtriert, und das Filtrat wird unter vermindertem Druck ein gedampft. Zum Entfernen der letzten Spuren von Wasser wird der Rückstand einige Male aus einer Mischung von Benzol und Aethanol eingedampft. Der erhaltene Festkörper wird mit 2,5 1 Aether angerieben, wobei man 159,7 g (2S)-3-[N-Mesyl-(1-methyläthyl)amino]-1,2-propandiol vom Schmelzpunkt 67-70° erhält. Einengen der Aetherlösung liefert weitere 27,7 g (2S)-3-[N-Mesyl-(1-methyläthyl)amino]-1,2-propandiol vom Schmelzpunkt 64-66°. Kristallisation aus Aethylacetat/Hexan liefert analytisch reines Material vom Schmelzpunkt 73-74°; $[\alpha]_D^{25}$ -15,94° (c = 1% in Wasser).

Analyse für $C_7H_{17}NO_4S$:
Berechnet: C, 39,79, H, 8,11, N, 6,63, S, 15,18
Gefunden: C, 39,83 H 8,40 N 6,66 S 14,96.

B.4) Eine Lösung von 211,3 g (2S)-3-[N-Mesyl-(1-methyl-äthyl)amino]-1,2-propandiol und 2,5 g Benzoesäure in 180 ml Trimethylorthoacetat wird in einem Gefäss, welches mit einer Vorrichtung zum Abdestillieren des während der Reaktion gebildeten Methanols versehen ist, auf 80-85° erhitzt. Nach 45 Minuten wird das Reaktionsgemisch abgekühlt und zwischen 600 ml Methylenchlorid und 600 ml einer 5%igen Natriumbicarbonatlösung verteilt. Die organische Phase wird mit verdünnter Natriumhydroxidlösung (2 x 200 ml) gewaschen, und die wässrige Phase wird mit 2 x 200 ml Methylenchlorid zurückgewaschen. Die vereinigten organischen Extrakte werden über Kaliumcarbonat getrocknet und eingedampft, wobei man 265 g gebildetes Orthoacetat als Oel erhält.

Dieses Oel wird in 500 ml Methylenchlorid gelöst und mit 150 ml Chlortrimethylsilan versetzt. Die Lösung wird während 45 Minuten zum Rückfluss erhitzt, danach abgekühlt und unter vermindertem Druck zur Trockene eingedampft, wobei man 268 g des Chloracetats erhält.

Dieses Material wird in 400 ml Methanol gelöst. Zur stark gerührten Lösung werden zunächst 200 ml Wasser und 200 g Eis gegeben. Danach wird innerhalb von 2-3 Minuten eine Lösung von 85 g Natriumhydroxid in 300 ml Wasser so zugegeben, dass die Reaktionstemperatur 15° nicht übersteigt. Nach 30-minütigem Rühren bei 15° wird der grösste Teil des Methanols unter vermindertem Druck abgedampft, und das verbleibende Gemisch wird mit 2 x 400 ml Methylenchlorid extrahiert. Die organischen Phasen werden mit 200 ml einer 5%igen Natriumchloridlösung gewaschen, danach kombiniert, getrocknet und eingedampft. Das zurückbleibende Oel wird unter vermindertem Druck destilliert, wobei man 182 g (S)-N-(1-Methyläthyl)-N-(methylsulfonyl)oxiran-methanamin vom Siedepunkt 118°/0,1 mm erhält;, $[\alpha]_D^{25}$ -20,06° (c = 1% in Methanol).

Analyse für $C_7H_{15}NO_3S$:
Berechnet: C, 43,50, H, 7,82, N, 7,25, S, 16,59
Gefunden: C, 43,26, H, 7,72, N, 7,29, S, 16,65.

B.5) Zu einer Lösung von 86 98 g (S)-N-(1-Methyläthyl)-N-(methylsulfonyl)oxiranmethanamin und 100 g 4-Benzyloxyphenol in 100 ml Methanol werden 5,04 g Kalium-tert.butoxid gegeben, und das Reaktionsgemisch wird während 16 Stunden unter Rückfluss gerührt. Danach werden 150 ml 2N Natriumhydroxidlösung zugegeben, wobei das Reaktionsgemisch zu einer festen Masse erstarrt. Diese wird mit 1 l 1N Natriumhydroxidlösung verdünnt und zum Digerieren des Festkörpers 1 Stunde gerührt. Danach wird der Festkörper abfiltriert und mit 1N Natriumhydroxidlösung und Wasser gewaschen. Das noch feuchte

Rohprodukt wird in Methylenchlorid gelöst, und die Lösung wird über Natriumsulfat getrocknet und unter vermindertem Druck partiell zu einem dicken Oel (250 g) eingedampft, welches danach unter Rühren mit 1 1 Aether verdünnt wird. Das Gemisch wird gekühlt, und das Produkt abfiltriert, wobei man 157,1 g (S)-1-(4-Benzyloxyphenoxy)-3-[N-mesyl-(1-methyläthyl)amino]-2-propanol vom Schmelzpunkt 96-97° erhält.

Analyse für $C_{20}H_{27}NO_5S$:
Berechnet: C, 61,04, H, 6,91, N, 3,56
Gefunden: C, 61,17, H, 6,90, N, 3,43.

B.6) Zu 78,7 g (S)-1-(4-Benzyloxyphenoxy)-3-[N-mesyl-(1-methyläthyl)amino]-2-propanol in 300 ml Toluol werden unter Rühren 29 ml Isopropenylmethyläther, gefolgt von 0,1 ml Phosphoroxychlorid, zugegeben. Das Reaktionsgemisch wird zur Bildung des Isopropenylmethylätherderivates während 2 Stunden bei Raumtemperatur gerührt. Darauf wird 1 ml Triäthylamin zur Neutralisation des Säurekatalysators zugegeben. Die Lösung wird dann tropfenweise während 30 Minuten zu einer gerührten Lösung von Natrium-bis-(2-methoxyäthoxy)aluminiumhydrid (70% in Benzol; 286 ml) und 300 ml Toluol gegeben, wobei während der ganzen Zeit der Zugabe die Temperatur auf 80° gehalten wird. Nach zusätzlichem Rühren während 2 Stunden bei 80° wird das Reaktionsgemisch abgekühlt und das überschüssige Reagens durch tropfenweise Zugabe von 30 ml 2N Natriumhydroxidlösung zerstört; sobald die Reaktion aufhört, werden noch 300 ml 2N Natriumhydroxidlösung zugegeben. Die beiden Phasen werden getrennt, und die organische Phase wird je zweimal mit 1N Natriumhydroxidlösung und gesättigter Kochsalzlösung gewaschen. Die Toluolphase wird dann mit 300 ml Aether verdünnt und mit 800 ml 0,5N Salzsäure extrahiert. Der Säureextrakt wird mit Aether gewaschen, und die organische Phase wird mit 100 ml 0,5N Salzsäure zurückgewaschen. Die gerührten wässrigen Extrakte werden mit 100 ml 10N Natriumhydroxidlösung basisch gestellt, und der erhaltene Festkörper wird abfiltriert und getrocknet. Kristallisation des Rohprodukts aus Aethylacetat/Hexan liefert 54,7 g (S)-1-(4-Benzyloxyphenoxy)-3-(1-methyl-äthyl)amino-2-propanol vom Schmelzpunkt 94-96°.

Analyse für $C_{19}H_{25}NO_3$:
Berechnet: C 72,35 H, 7,99, N, 4,44
Gefunden: C, 72,38, H, 8,16, N, 4,43.

B.7) Eine Lösung von 53,4 g (S)-1-(4-Benzyloxyphenoxy)-3-(1-methyläthyl)amino-2-propanol in 500 ml Methanol wird über 5 g 10%igem Palladium auf Kohle bei Raumtemperatur. und einem Druck von 1 Atmosphäre hydriert. Nach Aufnahme von 4,3 1 Wasserstoff hört die Wasserstoffaufnahme innerhalb von 40 Minuten abrupt auf. Das Reaktionsgemisch wird durch Celit filtriert, und das Filtrat wird unter vermindertem Druck zur Trockene eingedampft. Der zurückbleibende farblose feste Rückstand wird aus Aceton kristallisiert und liefert 35,2 g (S)-1-(4-Hydroxyphenoxy)-3-(1-methyläthyl)amino-2-propanol vom Schmelzpunkt 127-129°; $[\alpha]_D^{25}$ -20,67° (c = 1% in 0,1N Salzsäure).

Analyse für $C_{12}H_{19}NO_3$:
Berechnet: C 63,98 H 8,50, N, 6,22
Gefunden: C, 63,81, H, 8,68, N, 6,40.

C.1) Zu einer Lösung von 9,4 g des Dihydrochlorids von 1-[3-(2-Chlor-10H-phenothiazin-10-yl)propyl]-4-(2-chloräthyl)piperazin in 75 ml Dimethylsulfoxid werden 6 g (S)-1-(4-Hydroxyphenoxy)-3-(1-methyläthyl)amino-2-propanol gefolgt von 6,25 ml 4N Natriumhydroxidlösung gegeben. Das Gemisch wird unter Argon bei 55° während 2 Stunden gerührt, danach abgekühlt, mit 500 ml Wasser und 35 ml 1N Natriumhydroxidlösung verdünnt und dreimal mit Methylenchlorid extrahiert. Die Extrakte werden zweimal mit Wasser zurückgewaschen und über Kaliumcarbonat getrocknet. Das Lösungsmittel wird unter vermindertem Druck entfernt, wobei man 12,8 g rohes (S)-1-(1-Methyläthylamino)-3-[4-[2-[4-[3-(2-chlor-10H-phenothiazin-10-yl)propyl]piperazin-1-yl]äthoxy]phenoxy]-2-propanol als Oel erhält.

Das Oel wird in Methanol gelöst und mit überschüssiger methanolischer Salzsäure versetzt (ungefähr 6N; 12 ml). Die Lösung wird durch Kochen auf 70 ml eingeengt, dann gekühlt und danach mit 70 ml Aether bis zur Trübung versetzt. Das Gemisch wird gekühlt, und der erhaltene Festkörper wird abfiltriert. Auf diese Weise erhält man 13,4 g des Trihydrochlorids von (S)-1-(1-Methyläthylamino)-3-[4-[2-[4-[3-(2-chlor-10H-phenothiazin-10-yl)propyl]piperazin-1-yl]äthoxy]phenoxy]-2-propanol vom Schmelzpunkt 245-248°.

Umkristallisation dieses Materials aus Methanol/Aethylacetat liefert 11,0 g (S)-1-(1-Methyläthylamino)-3-[4-[2-[4-[3-(2-chlor-10H-phenothiazin-10-yl)propyl]piperazin-1-yl]äthoxy]phenoxy]-2-propanol-trihydrochlorid vom Schmelzpunkt 247-249°.

Das (S)-1-(1-Methyläthylamino)-3-[4-[2-[4-[3-(2-chlor-10H-phenothiazin-10-yl)propyl]piperazin-1-yl]äthoxy]-phenoxy]-2-propanol-trihydrochlorid kann auch wie folgt hergestellt werden:.

C.2) Zu einem Gemisch von 12,4 g des Dihydrochloridsalzes von 1-[3-(2-Chlor-10H-phenothiazin-10-yl)propyl]-4-(2-chloräthyl)piperazin und 6,2 g (S)-1-(4-Hydroxyphenoxy)-3-(1-methyläthyl)amino-2-propanol in 100 ml Dimethylsulfoxid werden 15,25 ml 5N Natriumhydroxidlösung gegeben, und das Gemisch wird unter Argon während 2 Stunden bei 50° gerührt. Das Reaktionsgemisch wird wie im Absatz C.1) oben aufgearbeitet, wobei man 14,7 g rohes (S)-1-(1-Methyläthyl-amino)-3-[4-[2-[4-[3-(2-chlor-10H-phenothiazin-10-yl)-propyl]piperazin-1-yl]äthoxy]phenoxy]-2-propanol erhält.

Dieses Produkt wird wie oben beschrieben in das Trihydrochloridsalz übergeführt (15,5 g Rohprodukt). Zweimaliges Umkristallisieren aus Methanol/Aethylacetat liefert 11,2 g des Trihydrochloridsalzes von (S)-1-(1-Methyläthyl-amino)-3-[4-[2-[4-[3-(2-chlor-10H-phenothiazin-10-yl)-propyl]piperazin-1-yl]äthoxy]phenoxy]-2-propanol vom Schmelzpunkt 251-253°; $[\alpha]_D^{25}$ -9,82° (c = 1% in Methanol).

Analyse für $C_{33}H_{43}ClN_4O_3S.3HCl$:

Berechnet: C, 55,00, H, 6,43, N, 7,77, Cl, 19,68, S, 4,44
Gefunden: C, 54,66, H, 6,44, N, 7,75, Cl, 19,60, S, 4,28.

**Beispiel 2**

A.1) Eine Lösung von 109 g 2(S)-Glycerin-2,3-acetonid welches nach der von LeCocq et al. in Biochem., 3, 976 (1964) beschriebenen Methode aus Mannitol-1,2,5,6-diacetonid via D-Glycerinaldehyd-acetonid erhalten wurde, in 1 l trockenem Methylenchlorid wird unter Rühren auf -35° gekühlt. Nach Zugabe von 140 ml Triäthylamin werden 71 ml Mesylchlorid innerhalb von 10 Minuten tropfenweise zugegeben, wobei man die Reaktionstemperatur zwischen -35 und -25° hält. Das Kühlbad wird entfernt, und nach 45 Minuten werden 250 ml 1N Salzsäure zugegeben. Die beiden Phasen werden getrennt, und die Methylenchloridlösung wird je einmal mit jeweils 250 ml-Portionen Wasser, 1N Natriumhydroxidlösung und nochmals Wasser gewaschen. Die wässrigen Phasen werden über Natriumsulfat getrocknet und eingedampft, wobei man 172,1 g (2R)-3-Mesyloxy-1,2-propan-diol-acetonid als leicht gelbes Oel erhält.

A.2) Eine Lösung von 39 g Natriumhydroxid in 200 ml Wasser wird zu einer gut gerührten Lösung von 172,1 g (2R)-3-Mesyloxy-1,2-propandiol-acetonid und 200 g 4-Benzyloxy-phenol in 1,5 l Dimethylsulfoxid gegeben. Danach wird das Reaktionsgemisch auf einem Dampfbad während 3 Stunden erhitzt. Die abgekühlte Lösung wird mit 1 l 1N Natriumhydroxidlösung verdünnt, und der erhaltene Festkörper wird mit verdünnter Natriumhydroxidlösung und Wasser gewaschen. Der luftgetrocknete Festkörper wird in 2 l Benzol aufgenommen, und die Lösung wird über Natriumsulfat getrocknet, mit Kohle entfärbt und unter vermindertem Druck eingedampft. Das Rohprodukt wird in 1,5 l heissem Methanol gelöst auf ungefähr 40° abgekühlt und von nicht-polaren Verunreinigungen abfiltriert. Ein kleiner Anteil der Methanollösung wird mit Wasser verdünnt, wobei man (2S)-3-(4-Benzyloxyphenoxy)-1,2-propandiol-acetonid erhält. Kristallisation aus Hexan liefert eine reine Probe vom Schmelzpunkt 69,5-71°; $[\alpha]_D^{25}$ +6,26° (c = 1% in Methanol).

Analyse für $C_{19}H_{22}O_4$:
Berechnet: C, 72,59, H, 7,05
Gefunden: C, 72,67, H, 6,90.

A.3) Die methanolische Lösung von (2S)-3-(4-Benzyloxy-phenoxy)-1,2-propandiol-acetonid (ungefähr 2 l) aus dem obigen Absatz wird mit 120 ml Wasser verdünnt und mit Dowex® 50W-8X-Ionenaustauscherharz (50 ml) in einem Gefäss versetzt, welches mit einer Destillationsvorrichtung versehen ist. Das gerührte Reaktionsgemisch wird zum Sieden erhitzt, und über einen Zeitraum von 3 Stunden werden 1,8 l Destillat abgetrennt. Das Reaktionsgemisch wird danach mit 500 ml Aethanol und 500 ml Benzol verdünnt, zur Entfernung des Harzes filtriert und unter vermindertem Druck zur Trockene eingedampft. Kristallisation des Rückstandes aus Aether und aus Aethylacetat liefert 137,0 g (2R)-3-(4-Benzyloxyphenoxy)-1,2-propandiol in verschiedenen Fraktionen.

Umkristallisation einer Probe aus Aethylacetat liefert analysenreines Material vom Schmelzpunkt 125-126,5°; $[\alpha]_D^{25}$ -5,52° (c = 1% in Methanol).

Analyse für $C_{16}H_{18}O_4$:
Berechnet: C 70,06, H, 6,61
Gefunden: C 70,01, H, 6,51.

A.4) Ein Gemisch von 137 g (2R)-3-(4-Benzyloxyphenoxy)-1,2-propandiol, 90 g Trimethylorthoacetat und 4 g Benzoesäure wird gerührt und während 45 Minuten auf 80° erhitzt, wobei man das gebildete Methanol aus dem Reaktionsgemisch abdestilliert. Das Reaktionsgemisch wird danach in 750 ml Benzol gegossen, und die Lösung wird mit 250 ml IN Natriumhydroxidlösung gewaschen. Die wässrige Phase wird mit Benzol zurückgewaschen, und die vereinigten organischen Extrakte werden über Kaliumcarbonat getrocknet und eingedampft, wobei man 166 g rohes Orthoacetat als Oel erhält. Das Oel wird in 500 ml trockenem Methylenchlorid gelöst mit 135 ml Chlortrimethylsilan versetzt und während 30 Minuten zum Rückfluss erhitzt. Das Lösungsmittel wird unter vermindertem Druck entfernt, wobei man 170 g Chloracetat erhält, welches man in 800 ml Methanol, welches 200 ml 2N methanolische Salzsäure enthält, löst. Das Gemisch wird über Nacht bei Raumtemperatur stehengelassen und danach unter vermindertem Druck zur Trockene eingedampft. Kristallisation des Rückstandes aus Methylenchlorid/Hexan liefert 128,9 g (2S)-1-Chlor-3-(4-benzyloxy-phenoxy)-2-propanol. Eine kleine Probe wird aus Aethylacetat umkristallisiert und liefert analyenreines Material vom Schmelzpunkt 78-80°; $[\alpha]_D^{25}$ +3,09° (c = 1% in Methanol).

Analyse für $C_{16}H_{17}O_3Cl$:
Berechnet: C 65,69, H, 5,85, C1, 12,11
Gefunden: C 65,43, H 5,74, Cl, 12,06.

A.5) 14,6 g (2S)-1-Chlor-3-(4-benzyloxyphenoxy)-2-propanol und 4,2 g Natriumacetat in 150 ml Methanol enthaltend 45 ml Isopropylamin, werden während 16 Stunden zum Ruckfluss erhitzt. Die Lösungsmittel werden unter vermindertem Druck entfernt, und der Rückstand wird zwischen 600 ml Benzol und 125 ml IN Natriumhydroxidlösung verteilt. Die wässrige Phase wird mit 200 ml Benzol gewaschen, und die organischen Phasen werden vereinigt, über Kaliumcarbonat getrocknet und unter vermindertem Druck eingedampft, wobei man rohes (2R)-1-(4-Benzyloxyphenoxy)-3-(1-methyläthyl)-amino-2-propanol als kristallinen Festkörper erhält. Kristallisation aus Aether liefert 13,7 g (2R)-1-(4-Benzyl-oxyphenoxy)-3-(1-methyläthyl)amino-2-propanol vom Schmelzpunkt 93-95°, und Umkristallisation aus Aether liefert -analysenreines Material vom Schmelzpunkt

93,5-94,5°; $[\alpha]_D^{25}$ +22,1° (c = 1% in 0,1N Salzsäure).
Analyse für $C_{19}H_{25}NO_3$:
Berechnet: C, 72,35, H, 7,99, N, 4,44
Gefunden: C, 72,40, H, 8,02, N, 4,29.

A.6) Hydrierung von (2R)-1-(4-Benzyloxyphenoxy)-3-(1-methyläthyl)amino-2-propanol unter den Reaktionsbedingungen wie in Beispiel 1, Absatz B.7) für die Herstellung von (S)-1-(4-Hydroxyphenoxy)-3-(1-methyläthylamino)-2-propanol angegeben, liefert das rechtsdrehende Isomere (R)-1-(4-Hydroxyphenoxy)-3-(1-methyläthyl)amino-2-propanol vom Schmelzpunkt 126-127°, $[\alpha]_D^{25}$ +20,85° (c = 1% in 0,1N Salzsäure).
Analyse für $C_{12}H_{19}NO_3$:
Berechnet: C 63,98,H8,50,N6,22
Gefunden: C, 64,04, H, 8,53, N, 6,04.

B) Unter den in Beispiel 1, Absatz C.1) angegebenen Reaktionsbedingungen werden 11,7 g 1-[3-(2-Chlor-10H-phenothiazin-10-yl)propyl]-4-(2-chloräthyl)piperazin mit 7,5 g (R)-1-(4-Hydroxyphenoxy)-3-(1-methyläthyl)amino-2-propanol zu (S)-1-(1-Methyläthylamino)-3-[4-[2-[4-[3-(2-chlor-10H-phenothiazin-10-yl)propyl]piperazin-1-yl]äthoxy]-phenoxy]-2-propanol-trihydrochlorid umgesetzt, welches nach Umkristallisation aus Methanol/Aethylacetat und aus Methanol/Aethanol bei 251,5-253° schmilzt. $[\alpha]_D^{25}$ +10,37° (c = 1% in Methanol).
Analyse für $C_{33}H_{43}ClN_4O_3S.3HC1$:
Berechnet: C, 55,00, H, 6,43, N, 7,77, Cl, 19 68 S, 4,44
Gefunden: C, 55,18, H, 6,50, N, 7,69, Cl, 19,35, S, 4,50.

**Beispiel 3**

A.1) Ein Gemisch von 50 g 4-Benzyloxyphenol 101 g 1,3-Dibrompropan und 17,3 g fein verriebenem wasserfreiem Kaliumcarbonat in 500 ml Aceton wird während 30 Stunden unter Rückfluss gerührt. Die Festkörper werden abfiltriert, und das Filtrat wird unter vermindertem Druck zur Trockene eingedampft. Der Rückstand wird in 300 ml Methylenchlorid gelöst, und die erhaltene Lösung wird zur Entfernung von nicht reagiertem 4-Benzyloxyphenol mit 10%iger Natriumhydroxidlösung gewaschen. Die Methylenchloridextrakte werden über Kaliumcarbonat getrocknet, unter vermindertem Druck eingeengt und destilliert, wobei man 33,4 g 1-Benzyloxy-4-(3-brompropoxy)benzol vom Siedepunkt 180-195°/0,6 mm erhält (41% Ausbeute, 98% Reinheit gemäss Gaschromatogramm). Die Struktur wird durch das Massenspektrum bestätigt.

A.2) In einem mit Glas ausgelegten Reaktionsgefäss werden 33,4 g 1-Benzyloxy-4-(3-brompropoxy)benzol in 300 ml Isopropylamin während 10 Stunden unter Stickstoff bei einem Druçk von 1000 psi auf 100° erhitzt. Danach wird das überschüssige Isopropylamin unter vermindertem Druck entfernt, und der Rückstand wird in 300 ml Wasser gelöst. Die Lösung wird mit 10%iger Natriumhydroxidlösung alkalisch gestellt, und das gebildete Oel wird mit Methylenchlorid extrahiert. Nach Trocknen über Kaliumcarbonat wird der Methylenchloridextrakt eingedampft und destilliert, wobei man 22,5 g 1-Benzyloxy-4-[3-(1-methyläthyl)aminopropoxy]benzol vom Siedepunkt 180-190°/0,5 mm erhält (97,8% rein gemäss Gaschromatogramm).

A.3) 11 5 g 1-Benzyloxy-4-[3-(1-methyläthyl)aminopropoxy]-benzol werden in 250 ml Aethanol über 1 g 10%igem Palladium auf Kohle bei Raumtemperatur und einem Anfangsdruck von 50 psi hydriert. Nach Aufnahme der theoretischen Menge an Wasserstoff wird das Reaktionsgemisch filtriert, und das Lösungsmittel wird unter vermindertem Druck abdestilliert, wobei man 8,1 g 4-[3-(1-Methyläthyl)amino-propoxy]phenol vom Schmelzpunkt 92-100° erhält (96,4% rein gemäss Gaschromatogramm; Massenspektrum übereinstimmend).

B) Unter den Reaktionsbedingungen, wie sie in Beispiel 1, Absatz C.1), beschrieben sind, werden 13,9 g 1-[3-(2-Chlor-10H-phenothiazin-10-yl)propyl]-4-(2-chloräthyl)piperazin in Gegenwart von 9,25 ml 4,0N Natriumhydroxidlösung in 100 ml Dimethylsuüfoxid mit 7,0 g 4-[3-(1-Methyläthyl)-aminopropoxy]phenol umgesetzt. Die rohe freie Base, welche durch Extraktion mit Chloroform isoliert wird, wird in Aethylacetat gelöst und mit Chlorwasserstoff in Aethylacetat zum rohen Trihydrochloridsalz umgesetzt. Kristallisation aus Methanol/Isopropanol liefert 10,8 g 1-[3-(2-Chlor-10H-phenothiazin-10-yl)propyl]-4-[2-[4-[3-(1-methyläthyl)aminopropoxy]phenoxy]äthyl]piperazin-trihydrochlorid vom Schmelzpunkt 215-225°.
Analyse für $C_{33}H_{43}ClN_4O_2S.3HC1$:
Berechnet: C, 56,25, H, 6,58, N; 7,95
Gefunden: C, 56,59, H, 6,48 N 7,78.

**Beispiel 4**

A.1) 4-Benzyloxyphenol wird mit 1,4-Dibrompropan unter den im wesentlich gleichen Reaktionsbedingungen wie in Beispiel 3, Absatz A.1) beschrieben umgesetzt, ausgenommen dass die Reaktionsdauer 118 Stunden beträgt und das Lösungsmittel für die Extraktion Toluol ist. Das Rohprodukt wird aus Hexan kristallisiert und liefert 1-Benzyloxy-4-(4-brombutoxy)benzol vom Schmelzpunkt 70-72°.

Analyse für $C_{17}H_{19}BrO_2$:
Berechnet: C 60,91 H 5,71
Gefunden: C, 61,36, H, 5,78.

A.2) Wie in Beispiel 3, Absatz A.2) beschrieben wird das 1-Benzyloxy-4-(4-brombutoxy)benzol bei 100° und einem Druck von 500 psi während 12 Stunden mit Isopropylamin zum 1-Benzyloxy-4-[4-(1-methyläthyl)aminobutoxy]benzol umgesetzt. Das Rohprodukt wird nicht destilliert.

A.3) 1-Benzyloxy-4-[4-(1-methyläthyl)aminobutoxy]benzol wird unter den Hydrierungsbedingungen wie in Beispiel 3, Absatz A.3) beschrieben debenzyliert. Das Rohprodukt wird aus Cyclohexan kristallisiert und liefert 4-[4-(1-Methyl-äthyl)aminobutoxy]phenol vom Schmelzpunkt 91-93° (98,6% rein gemäss Gaschromatogramm).

B) Eine Lösung von 3,8 g 1-[3-(2-Chlor-10H-phenothiazin-10-yl)propyl]-4-(2-chloräthyl)piperazin n und 2 0 g 4-[4-(1-Methyläthyl)aminobutoxy]phenol in 100 ml Dimethylsulfoxid wird mit 0,4 g Natriumhydroxid in 3 ml Wasser unter den in Beispiel 1, Absatz C.1) angegebenen Reaktionsbedingungen umgesetzt. Das rohe Amin wird mit Aethylacetat extrahiert, und der getrocknete Extrakt wird durch Zugabe von überschüssigem Chlorwasserstoff in Aethylacetat in das entsprechende Trihydrochloridsalz übergeführt. Das rohe Salze wird mit einer kleinen Menge heissem Methanol angerieben und danach aus Methanol/Aethylacetat kristallisiert, wobei man 2,0 g 1-[3-(2-Chlor-10H-phenothiazin-10-yl)propyl]-4-[2-[4-[4-(1-methyläthyl)aminobutoxy]phenoxy]äthyl]piperazin-trihydrochlorid vom Schmelzpunkt 220-221° erhält.

Analyse für $C_{34}H_{45}ClN_4O_2S.3HCl$:
Berechnet: C, 56,82 H 6,73 N 7,80
Gefunden: C, 56,95, H, 6,59, N, 7,78.

**Beispiel 5**

A.1) 4-Benzyloxyphenol und 1,5-Dibrompentan werden unter den in Beispiel 3, Absatz A.1) beschriebenen Reaktionsbedingungen während 5 Tagen umgesetzt. Das Rohprodukt wird durch Extraktion mit Toluol isoliert und danach destilliert, wobei man 1-Benzyloxy-4-(5-brompentoxy)benzol als Oel erhält (88,9% rein gemäss Gaschromatogramm).

A.2) Wie in Beispiel 3, Absatz A.2) beschrieben wird das 1-Benzyloxy-4-(5-brompentoxy)benzol bei 100° und einem Druck von 150 psi während 12 Stunden mit Isopropylamin umgesetzt, wobei man 1-Benzyloxy-4-[5-(1-methyläthyl)aminopentoxy]benzol erhält. Das destillierte Produkt ist gemäss Gaschromatogramm 95,2% rein.

A.3) Eine Lösung von 24 g 1-Benzyloxy-4-[5-(1-methyläthyl)-aminopentoxy]benzol in 250 ml Essigsäure wird über 1 g 10% Palladium auf Kohle hydriert. Die theoretische Wasserstoffaufnahme ist nach halbstündigem Schütteln bei Raumtemperatur beendet. Das Lösungsmittel wird durch Destillation unter vermindertem Druck entfernt, und der Rückstand wird in Wasser gelöst. Die Lösung wird mittels gesättigter Natriumbicarbonatlösung basisch gestellt, und das Produkt wird abfiltriert, wobei man 13,0 g (75%) 4-[5-(1-Methyläthyl)aminopentoxy]phenol vom Schmelzpunkt 95-99° erhält (95 7% rein gemäss Gaschromatogramm). Das Massenspektrum ist mit dem erwarteten Produkt vereinbar.

B) Eine Lösung von 9,2 g 1-[3-(2-Chlor-10H-phenothiazin-10-yl)propyl]-4-(2-chloräthyl)piperazin und 5 16 g 4-[5-(1-Methyläthyl)aminopentoxy]phenol in 150 ml Dimethülsulfoxid wird mit 0,87 g Natriumhydroxid in 7 ml Wasser versetzt und während 5 Stunden bei 55-60° gerührt. Das übliche Aufarbeiten liefert das rohe Trihydrochloridsalz, welches aus Methanol umkristallisiert wird, wobei man 5,8 g 1-[3-(2-Chlor-10H-phenothiazin-10-yl)propyl]-4-[2-[4-[5-(1-methyläthyl)aminopentoxy]phenoxy]äthyl]piperazin-tri-hydrochlorid vom Schmelzpunkt 237-239° erhält.

Analyse für $C_{35}H_{47}ClN_4O_2S.3HC1$:
Berechnet: C, 57,38, H, 6,88, N, 7,65
Gefunden: C, 57,09, H, 6,98, N, 7,50.

**Beispiel 6**

A.1) 4-Benzyloxyphenol und 1,6-Dibromhexan werden unter den in Beispiel 3, Absatz A.1) beschriebenen Reaktionsbedingungen umgesetzt. Die Reaktionszeit beträgt 4 Tage,und die Extraktion wird mittels Toluol durchgeführt. Kristallisation des Rohproduktes aus Hexan liefert 1-Benzyloxy-4-(6-bromhexyloxy)benzol vom Schmelzpunkt 72-73°.

A.2) Wie in Beispiel 3, Absatz A.2) beschrieben wird das 1-Benzyloxy-4-(6-bromhexyloxy)benzol bei 100° und einem Druck von 500 psi während 12 Stunden mit Isopropylamin umgesetzt, wobei man 1-Benzyloxy-4-[6-(1-methyläthyl)aminohexyloxy]benzol erhält. Das rohe Oel kristallisiert aus Pentan und liefert reines Produkt vom Schmelzpunkt 47-49°.

A.3) Eine Lösung des rohen 1-Benzyloxy-4-[6-(1-methyläthyl)-aminohexyloxy]benzolswird unter den in Beispiel 3, Absatz A.3) für die Herstellung von 4-[3-(1-Methyläthyl)amino-propoxy]phenol beschriebenen Reaktionsbedingungen hydriert. Kristallisation einer kleinen Probe des Rohprodukts vom Schmelzpunkt 78-86° aus Cyclohexan liefert analytisch reines 4-[6-(1-Methyläthyl)aminohexyloxy]phenol vom Schmelzpunkt 90-92°.

Analyse für $C_{15}H_{25}NO_2$:
Berechnet: C 71 67 H, 10 03, N, 5,57
Gefunden: C, 71,50, H, 9,93, N, 5.64.

B) Eine Lösung von 10,8 g 1-[3-(2-Chlor-10H-phenothiazin-10-yl)propyl]-4-(2-chloräthyl)piperazin und 6,3 g 4-[6-(1-Methyläthyl)aminohexyloxy]phenol in 125 ml Dimethylsulfoxid wird mit 1,0 g Natriumhydroxid in 7 ml Wasser versetzt. Die Reaktion wird wie weiter oben beschrieben durchgeführt (1 Stunde; 55°), und das Reaktionsgemisch wird in der üblichen Weise aufgearbeitet (Extraktion mit Aethylacetat und Ausfällen des Salzes mittels Chlorwasserstoff in Aethylacetat). Kristallisation des rohen Trihydrochloridsalzes aus Methanol liefert 5,1 g 1-[3-(2-Chlor-10H-phenothiazin-10-yl)-4-[2-[4-[6-(1-methyläthyl)aminohexyl-oxy]phenoxy]äthyl]piperazin-trihydrochlorid vom Schmelzpunkt 199-203°.

Analyse für $C_{36}H_{49}ClN_4O_2S.3HC1$:
Berechnet: C, 57,91, H, 7,02, N, 7,50
Gefunden: C, 57,99, H, 7,15, N, 7,23.

**Beispiel 7**

A.1) Zu 150 ml Aethanol, welches 16 g Methylamin enthält, werden 14 g 1-Benzyloxy-4-(3-brompropyloxy)benzol gegeben. Die Lösung wird während 12 Stunden unter Stickstoff und einem Druck von 1000 psi auf 100° erhitzt. Das Lösungsmittel wird unter vermindertem Druck abgedampft, und der Rückstand wird mit 20%iger Natriumhydroxidlösung auf etwa pH 10 eingestellt. Das Oel wird mit Aethylacetat extrahiert und in der üblichen Weise in das entsprechende Hydrochloridsalz übergeführt. Auf diese Weise erhalt man 8,4 g (70%) 1-Benzyloxy-4-(3-methylaminopropoxy)benzol-hydrochlorid vom Schmelzpunkt 202-204°.

Analyse für $C_{17}H_{21}NO_2.HCl$:
Berechnet: C, 66,33, H, 7,20, N, 4,55
Gefunden: C 66 30, H, 7,08, N, 4,60.

A.2) Eine Lösung von 8,4 g 1-Benzyloxy-4-(3-methylamino-propoxy)benzol-hydrochlorid in 250 ml Essigsäure wird über 0 5 g 10% Palladium auf Kohle in einer Parr-Apparatur hydriert (50°; Anfangsdruck 50 psi). Die Reaktion ist innerhalb 0,4 Stunden beendet, und nach Abfiltrieren des Katalysators wird das Lösungsmittel unter vermindertem Druck abgedampft. Kristallisation des Rückstandes aus Methanol/Aethylacetat liefert 4,8 g (80%) 4-(3-Methylamino-propoxy)phenol-hydrochlorid vom Schmelzpunkt 167-169°.

Analyse für $C_{10}H_{15}NO_2.HC1$:
Berechnet: C, 55,17, H, 7,41, N, 6,43
Gefunden: C, 54,97, H, 7.42, N, 6,60.

B) Eine Lösung von 4,7 g 4-(3-Methylaminopropoxy)phenol-hydrochlorid und 10,7 g 1-[3-(2-Chlor-10H-phenothiazin-10-yl)propyl]-4-(2-chloräthyl)piperazin (in Form des Dihydrochlorids) in 125 ml Dimethylsulfoxid wird unter Rühren und unter Stickstoff mit 3,46 g Natriumhydroxid in 5 ml Wasser versetzt. Danach wird das Reaktionsgemisch während 4 Stunden bei 55° gerührt und anschliessend mit 500 ml Wasser verdünnt. Das ausgeschiedene Oel wird mit Aethylacetat extrahiert. Nach dem Trocknen des Extraktes über Kaliumcarbonat bläst man zum Ausfällen des Rohprodukts als Trihydrochloridsalz (4,3 g) Chlorwasserstoff in die Lösung. Kristallisation des Salzes aus Acetonitril/Methanol liefert 1,9 g 3-[[4-[2-[4-[3-(2-Chlor-10H-phenothiazin-10-yl)propyl]-1-piperazinyl]äthoxy]phenyl]oxy]-N-methylpropan-amin-trihydrochlorid vom Schmelzpunkt 223-226°.

Analyse für $C_{31}H_{39}ClN_4O_2S.3HC1$:
Berechnet: C, 55,03, H, 6,26, N, 8,28
Gefunden: C, 54,96, H, 6,27, N, 8,23.

**Beispiel 8**

A.1) In der in Beispiel 7, Absatz A.1) für die Herstellung von 1-Benzyloxy-4-(3-methylaminopropoxy)benzol angegebenen Weise wird 1-Benzyloxy-4-(3-brompropyloxy)benzol mit Dimethylamin zu 1-Benzyloxy-4-(3-dimethylaminopropoxy)benzol umgesetzt. Das Produkt wird in Form des Hydrochloridsalzes isoliert und aus Isopropanol kristallisiert, wobei man das reine Hydrochloridsalz von 1-Benzyloxy-4-(3-dimethyl-aminopropoxy)benzol vom Schmelzpunkt 183-185° erhält.

A.2) In der in Beispiel 7, Absatz A.2) für die Herstellung von 4-(3-Methylaminopropoxy)phenol-hydrochlorid beschriebenen Weise wird das 1-Benzyloxy-4-(3-dimethylaminopro-poxy)benzol in das 4-(3-Dimethylaminopropoxy)phenol-hydrochlorid übergeführt, wobei jedoch Aethanol als Lösungsmittel verwendet wird. Das Rohprodukt liefert nach Kristallisation aus Aethanol reines 4-(3-Dimethylaminopropoxy)phenol-hydrochlorid vom Schmelzpunkt 188-190°.

B) Eine Lösung von 4,22 g 1-[3-(2-Chlor-10H-phenothiazin-10-yl)propyl]-4-(2-chloräthyl)piperazin und 2,3 g 4-(3-Dimethylaminopropoxy)phenol-hydrochlorid in 100 ml Dimethylsulfoxid wird unter Stickstoff und Rühren auf 55° erwärmt. Danach wird eine Lösung von 0,8 g Natriumhydroxid in 3 ml Wasser zugegeben, und das Reaktionsgemischwird während 2 Stunden bei 55-65° gerührt. Uebliches Aufarbeiten liefert das rohe Salz,

welches aus Methanol/Aethylacetat kristallisiert wird, wobei man 1,5 g 3-[[4-[2-[4-[3-(2-Chlor-10H-phenothiazin-10-yl)propyl]-1-piperazinyl]äthoxy]phenyl]-oxy]-N,N-dimethylpropanamin-trihydrochlorid vom Schmelzpunkt 228-230° erhält.

**Beispiel 9**

A.1) Eine Lösung von 29,4 g 1-Benzyloxy-4-(3-brompropyloxy)-benzol und 20,4 g Kaliumphthalimid in 150 ml Dimethylformamid wird unter Stickstoff gerührt und auf einem Dampfbad während 17 Stunden erhitzt. Das Lösungsmittel wird unter vermindertem Druck abgedampft, und 250 ml Wasser werden zum Rückstand gegeben. Das Gemisch wird mit Methylenchlorid extrahiert, und der Extrakt wird über wasserfreiem Kaliumcarbonat getrocknet. Das Lösungsmittel wird unter vermindertem Druck abgedampft, und der Rückstand wird aus Aethanol/Aceton kristallisiert, wobei man 10 9 g (31%) 1-Benzyloxy-4-(3-phthalimidopropoxy)benzol vom Schmelzpunkt 143-145° erhält. Eine zweite Fraktion von 9,2 g (26%) und einem Schmelzpunkt von 137-140° erhält man durch Einengen des Filtrats. Die Analyse einer Probe vom Schmelzpunkt 141-143° aus einem früheren Ansatz ist wie folgt:

Analyse für $C_{24}H_{21}NO_4$:
Berechnet: C, 74,40, H, 5,46, N, 3,62
Gefunden: C, 74,58, H, 5,58, N, 3,45.

A.2) Ein Hydrierkolben, welcher 0,5 g 10% Palladium auf Kohle und 250 ml Essigsäure enthält, wird mit 16 g 1-Benzyl-oxy-4-(3-phthalimidopropoxy)benzol beladen. Das Gemisch wird während etwa 4 Stunden bei 50° in einer Parr-Hydrierungsapparatur geschüttelt und auf Raumtemperatur abgekühlt. Das Reaktionsgemisch wird filtriert, und das Lösungsmittel wird unter vermindertem Druck abgedampft. Kristallisation des Rückstandes aus Toluol liefert 10 8 g (88%) 4-(3-Phthalimidopropoxy)phenol vom Schmelzpunkt 135-137°.

Analyse für $C_{17}H_{15}NO_4$:
Berechnet: C, 68,68, H, 5,09, N, 4,71
Gefunden: C, 68,64, H, 5,22, N, 4,33.

B.1) Zu einer gerührten Lösung von 24 g 1-[3-(2-Chlor-10H-phenothiazin-10-yl)propyl]-4-(2-chloräthyl)piperazin und 14,5 g 4-(3-Phthalimidopropyloxy)phenol in 500 ml Dimethylsulfoxid werden bei 50° unter Stickstoff 1,9 g Natriumhydroxid in 15 ml Wasser gegeben, und die Lösung wird danach während 5 Stunden bei 55-60° gerührt. Nachdem man über Nacht bei Raumtemperatur stehengelassen hat, wird das Reaktionsgemisch in 1 l Wasser gegossen und das Oel mit Aethylacetat extrahiert. Die Extrakte werden nach dem Trocknen über Natriumsulfat unter vermindertem Druck eingedampft, und der Rückstand wird in Acetonitril gelöst. Nach Abdekantieren vom unlöslichen Material wird Chlorwasserstoff in die Lösung geblasen, um das Produkt als Dihydrochloridsalz auszufällen. Danach gibt man Methanol zu, um das Salz wieder zu lösen, und engt die Lösung bis zum Beginn eines Ausfalls ein. Der Festkörper wird abfiltriert, wobei man 2 g 1-[3-(2-Chlor-10H-phenothiazin-10-yl)propyl]-4-[2-[4-(3-phthalimidopropyloxy)phenoxy]äthyl]-piperazin als Dihydrochloridsalz vom Schmelzpunkt 233-246° erhält. Das Filtrat wird eingedampft, und das verbleibende Oel wird aus Methanol kristallisiert, wobei man weitere 8,3 g des gleichen Produkts vom Schmelzpunkt 225-240° erhält.

B.2) Zu 10,5 g 1-[3-(2-Chlor-10H-phenothiazin-10-yl)propyl]-4-[2-[4-(3-phthalimidopropyloxy)phenoxy]äthyl]piperazin in 500 ml Aethanol werden 2,95 g 85%iges Hydrazinhydrat gegeben. Die Lösung wird während 24 Stunden unter Rühren zum Rückfluss erhitzt. Das unlösliche Phthalazindion wird danach abfiltriert, und das Filtrat wird unter vermindertem Druck eingeengt. 200 ml Wasser und 50 ml konzentrierte Ammoniumhydroxidlösung werden zum Rückstand gegeben. Das Oel wird mit Methylenchlorid extrahiert, und der Extrakt wird über wasserfreiem Natriumsulfat getrocknet. Das Lösungsmittel wird unter vermindertem Druck entfernt, und der Rückstand wird in Aethylacetat gelöst. Um das Produkt als Trihydrochloridsalz auszufällen bläst man Chlorwasserstoff in die Lösung. Das Salz wird abfiltriert und aus Acetonitril/Methanol kristallisiert. Die Ausbeute an 3-[[4-[2-[4-[3-(2°Chlor-10H-phenothiazin-10-yl)propyl]-1-pipera-zinyl]äthoxy]phenyl]oxy]propanamin-trihydrochlorid vom Schmelzpunkt 237-240° beträgt 6 0 g.

Analyse für $C_{30}H_{37}ClN_4O_2S.3HCl$:
Berechnet: C, 54,39, H, 6,09, N, 8,46
Gefunden: C, 54,06, H, 6,03, N, 8,18.

**Beispiel 10**

A) Ein Gemisch von 300 ml Isopropanol und 17,2 g 4-(2-Eromäthyl)phenol wird in einem mit Glas ausgelegten Reaktionsgefäss während 12 Stunden unter Stickstoff und einem Anfangsdruck von 250 psi auf 100° erhitzt. Das Lösungsmittel wird unter vermindertem Druck entfernt, und der Rückstand wird unter Verwendung von 10%iger Natriumcarbonatlösung basisch gestellt. Das Gemisch wird mit Aether extrahiert, und die Aetherlösung wird nach Trocknen über Kaliumcarbonat unter vermindertem Druck eingedampft. Der

verbleibende Rückstand kristallisiert beim Stehenlassen und liefert 13,5 g 4-[2-(1-Methyläthyl)aminoäthyl]phenol (98% rein gemäss Gaschromatogramm). Das Massenspektrum ist mit dem erwarteten Produkt vereinbar. Eine kleine Probe wird aus Cyclohexan/Benzol kristallisiert und schmilzt bei 102-104°.

B) Eine Lösung von 10 g 1-[3-(2-Chlor-10H-phenothiazin-10-yl)propyl-4-(2-chloräthyl)piperazin und 4,22 g 4-[2-(1-Methyläthyl)aminoäthyl]phenol in 100 ml Dimethylsulfoxid wird unter Stickstoff und Rühren auf 50° erwärmt. Eine Lösung von 1 g Natriumhydroxid in 7 ml Wasser wird zugegeben. Die erhaltene Lösung wird danach während 5 Stunden auf 55-60° erhitzt. Zum abgekühlten Reaktionsgemisch werden 200 ml Wasser gegeben, und das Oel wird mit Methylenchlorid extrahiert. Nach Trocknen über Kaliumcarbonat wird die Lösung unter vermindertem Druck eingedampft und der Rückstand in das Trihydrochloridsalz übergeführt. Kristallisation des rohen Salzes aus Methanol/Aethylacetat liefert 3,9 g eines Produkts vom Schmelzpunkt 244-246°. Dieses Salz wird in Wasser gelöst und durch Zugabe von Kaliumcarbonat in die freie Base übergeführt. Das Oel wird mit Aethylacetat extrahiert, und die Losung wird wie oben getrocknet. Das Lösungsmittel wird unter vermindertem Druck abdestilliert, wobei man 3 g freie Base erhält. Eine Lösung dieser freien Base in 10 ml Aethanol wird zu einer Lösung von 1,85 g Maleinsäure in 10 ml Aethanol gegeben. Abfiltrieren des erhaltenen kristallinen Festkörpers liefert 4 g 1-[3-(2-Chlor-10H-phenothiazin-10-yl)propyl]-4-[2-[4-[2-(1-methyläthyl)aminoäthyl]phenoxy]äthyl]piperazintrimaleat vom Schmelzpunkt 178-180°.

Analyse für $C_{32}H_{41}ClN_4OS.3C_4H_4O_4$:
Berechnet: C, 57,86, H, 5,85, N, 6,13
Gefunden: C, 58,05, H, 5,91, N, 5,94.

**Beispiel 11**

A.1) Man gibt zu 110 g einer 48%igen Lösung von Bromwasserstoff 25 g 4-(3-Hydroxypropyl)phenol und erhitzt das Gemisch während 3 Stunden auf einem Dampfbad. Nach dem Abkühlen gibt man 500 ml Wasser zu, extrahiert das Oel mit Aether, wäscht mit Wasser und verdünnt danach mit Natriumbicarbonatlösung. Der Aetherextrakt wird über Kaliumcarbonat getrocknet und eingedampft. Der Rückstand wird in einem Kugelrohr destilliert, wobei man 29,6 g (84%) 4-(3-Brompropyl)phenol vom Siedepunkt 180°/2 mm erhält. Das Produkt ist gemäss Gaschromatogramm 92,6% rein, und das Massenspektrum stimmt mit 4-(3-Brompropyl)phenol überein.

A.2) Unter den in Beispiel 10, Absatz A) für die Herstellung von 4-[2-(1-Methyläthyl)aminoäthyl]phenol angegebenen Reaktionsbedingungen wird 4-(3-Brompropyl)phenol mit Isopropylamin umgesetzt. Das Rohprodukt wird in einem Kugelrohr destilliert (150°/1 mm), und das Destillat wird aus Aether kristallisiert, wobei man 4-[3-(1-Methyläthyl)aminopropyl]-phenol erhält (97,9% rein gemäss Gaschromatogramm).

B) Eine Lösung von 10 g 1-[3-(2-Chlor-10H-phenothia-zin-10-yl)propyl]-4-(2-chloräthyl)piperazin und 4,5 g 4-[3-(1-Methyläthyl)aminopropyl]phenol in 100 ml Dimethylsulfoxid, welches 0,94 g Natriumhydroxid in 7 ml Wasser enthält, wird während 5 Stunden bei 55-60° gerührt. Das rohe Trihydrochloridsalz, welches nach dem üblichen Aufarbeiten erhalten wird, wird aus Methanol/Acetonitril kristallisiert, wobei man 4,5 g 1-[3-(2-Chlor-10H-pheno-thiazin-10-yl)propyl]-4-[2-[4-[3-(1-methyläthyl)amino-propyl]phenoxy]äthyl]piperazin-trihydrochlorid vom Schmelzpunkt 216-218° erhält.

Analyse für $C_{33}H_{43}ClN_4OS.3HCl$:
Berechnet: C, 57,56, H, 6,73, N, 8,14
Gefunden: C, 57,25, H, 6,94, N, 7,93.

**Beispiel 12**

A.1) Eine Lösung von 13,7 g 4-(4-Methoxyphenyl)butylbromid in 50 ml Isopropylamin wird in einem mit Glas ausgelegten Gefäss bei einem Anfangsdruck von 500 psi unter Stickstoff während 12 Stunden auf 100° erhitzt. Man entfernt das Lösungsmittel unter vermindertem Druck und gibt zum Rückstand 200 ml Wasser. Die Lösung wird mit konzentrierter Salzsäure angesäuert und mit Aether extrahiert. Der Extrakt wird über Kaliumcarbonat getrocknet, und das Lösungsmittel wird unter vermindertem Druck entfernt, wobei man 9 3 g (75%) 4-(4-Methoxyphenyl)-N-(1-methyläthyl)butylamin erhält. Die Identität und Reinheit (94,8%) des Produkts wird mittels Massenspektrum und Gaschromatographie bestimmt.

A.2) 10 g 4-[4-Methoxyphenyl]-N-(1-methyläthyl)butylamin werden zu 70 ml einer 48%igen Lösung von Bromwasserstoff gegeben, und das Reaktionsgemisch wird während 3 Stunden zum Rückfluss erhitzt. Der verbleibende Bromwasserstoff wird unter vermindertem Druck abdestilliert, und der Rückstand wird mit 10%iger Natriumcarbonatlösung alkalisch gestellt. Das Produkt wird abfiltriert, mit Wasser gewaschen und getrocknet, wobei man 5,0 g 4-[4-(1-Methyläthyl)aminobutyl]-phenol erhält.. Massenspektrum und Gaschromatographie bestätigen die Identität und Reinheit (93,5%) des Produkts.

B) Eine Lösung von 10 g 1-[3-(2-Chlor-10H-phenothiazin-10-yl)propyl]-4-(2-chloräthyl)piperazin und 4,9 g 4-[4-(1-Methyläthyl)aminobutyl]phenol wird unter Stickstoff bei 55° gerührt, und eine Lösung von 0,94 g Natriumhydroxid in 7 ml Wasser wird zugegeben. Das Reaktionsgemisch wird während 5 Stunden unter

Rühren auf 55-65° erhitzt. Uebliches Aufarbeiten liefert rohes Trihydrochloridsalz. Kristallisation des Produkts aus Methanol/Aethanol liefert 5 3 g 1-[3-(2-Chlor-10H-phenothiazin-10-yl)propyl]-4-[2-[4-[4-(1-methyläthyl)aminobutyl]phenoxy]äthyl]piperazin-trihydrochlorid vom Schmelzpunkt 245-248°.

Analyse für $C_{34}H_{45}ClN_4OS.3HCl$:
Berechnet: C, 58,12, H, 6,89, N, 7,97
Gefunden: C, 57,88, H, 6,83, N, 7,85.

**Beispiel 13**

A) 41 g 4-(2-Bromäthoxy)phenol werden zu 300 ml Isopropylamin gegeben, und die Lösung wird während 12 Stunden unter Stickstoff bei einem Druck von 250 psi auf 100° erhitzt. Das überschüssige Isopropylamin wird unter vermindertem Druck entfernt, und 300 ml Wasser werden zum Lösen des Rückstandes zugegeben. Die Lösung wird mit festem Kaliumcarbonat basisch gestellt, und das Oel wird mit Aether extrahiert. Der Aetherextrakt wird über wasserfreiem Kaliumcarbonat getrocknet. Nach dem Entfernen des Lösungsmittels unter vermindertem Druck wird der Rückstand aus Aceton/Hexan umkristallisiert, wobei man 21,8 g (59%) 4-[2-(1-Methyläthyl)aminoäthoxy]phenol vom Schmelzpunkt 78-80° erhält.

Analyse für $C_{11}H_{17}NO_2$:
Berechnet: C, 67,66, H, 8,78, N, 7,17
Gefunden: C, 67,57, H, 9,06, N, 7.25.

B) Unter den im wesentlichen gleichen Reaktionsbedingungen wie in Beispiel 1, Absatz C.1) oben beschrieben werden 10,6 g 1-[3-(2-Chlor-10-phenothiazin-10-yl)propyl]-4-(2-chloräthyl)piperazin mit 4,88 g 4-[2-(1-Methyläthyl)amino-äthoxy]phenol in 125 ml Dimethylsulfoxid, welches 1 g Natriumhydroxid in 7 ml Wasser enthält, umgesetzt. Die rohe freie Base wird mit Aethylacetat extrahiert, und die getrockneten Extrakte werden zur Ausfällung des rohen Trihydrochloridsalzes mit überschüssigem Chlorwasserstoff in Aethylacetat versetzt. Kristallisation des rohen Salzes aus Methanol/Isopropanol liefert 2,5 g 1-[3-(2-Chlor-10H-phenothiazin-10-yl)propyl]-4-[2-[4-[2-(1-methyläthyl)amino-äthoxy]phenoxy]äthyl]piperazin-trihydrochlorid vom Schmelzpunkt 223-235°.

Analyse für $C_{32}H_{41}ClN_4O_2S.3HCl$:
Berechnet: C, 55,65, H, 6,42, N, 8,11
Gefunden: C, 55,61, H, 6,56, N, 7,97.

**Beispiel 14**

A) 4-[3-[2-(Trifluormethyl)phenothiazin-10-yl]propyl]-1-piperazinäthanol wird wie in Beispiel 1, Absatz A) beschrieben in die entsprechende Chloräthylverbindung übergeführt, wobei das 1-[3-(2-Trifluormethyl-10H-phenothiazin-10-yl)propyl]-4-(2-chloräthyl)piperazin als Oel anfällt.

B) 4,8 g 1-[3-(2-Trifluormethyl-10H-phenothiazin-10-yl)-propyl]-4-(2-chloräthyl)piperazin und 2,84 g (S)-1-(4-Hydroxyphenoxy)-3-(1-methyläthyl)amino-2-propanol in 100 ml Dimethylsulfoxid werden mit 2,9 ml einer 4N Natriumhydroxidlösung erhitzt und während 1 Stunde bei 55° gehalten. Uebliches Aufarbeiten liefert 6,4 g der rohen freien Base.

6,0 g der Base werden unter Verwendung von methanolischer Salzsäure in das entsprechende Trihydrochlorid übergeführt. Zweimalige Umkristallisation des Salzes aus Methanol/Aethylacetat liefert 4,03 g (S)-1-(1-Methyläthylamino)-3-[4-[2-[4-[3-(2-trifluormethyl-10H-phenothiazin-10-yl)-propyl]piperazin-1-yl]äthoxy]phenoxy]-2-propanol-trihydro-chlorid vom Schmelzpunkt 242-244°; $[\alpha]_D^{25}$ -7,59° (c = 1% in Methanol).

Analyse für $C_{34}H_{43}F_3N_4O_3S.3HCl$:
Berechnet: C, 54,15, H, 6,15, N, 7,43, F, 7,56, Cl, 14,10, S, 4,25
Gefunden: C, 53,96, H, 6,17, N, 7,28, F, 7,31, Cl, 14,06, S, 4,38.

**Beispiel 15**

A) 4-[3-(2-Methylthio-10H-phenothiazin-10-yl)propyl]-1-piperazinäthanol wird in Beispiel 1, Absatz A) in die entsprechende Chloräthylverbindung überführt, wobei das 1-[3-(2-Methylthio-10H-phenothiazin-10-yl)propyl]-4-(2-chloräthyl)piperazin als Oel anfällt.

B) Eine Lösung von 3,2 g 1-[3-(2-Methylthio-10H-pheno-thiazin-10-yl)propyl]-4-(2-chloräthyl)piperazin und 2 0 g 4-[5-(1-Methyläthyl)aminopentoxy]phenol in 50 ml Dimethylsulfoxid wird mit einer Lösung von 0,3 g Natriumhydroxid in 2 ml Wasser versetzt. Das Gemisch wird während 5 Stunden auf 55-60° erwärmt und danach in üblicher Weise aufgearbeitet. Das Produkt wird in Form seines Trimaleatsalzes isoliert, welches durch Kristallisation aus Methanol/Aethylacetat gereinigt wird. Das so erhaltene 1-[3-(2-Methylthio-10H-phenothiazin-10-yl)propyl]-4-[2-[4-[5-(1-methyläthyl)aminopentoxy]phenoxy]äthyl]piperazin-trimaleat schmilzt bei 136-138°.

Analyse für $C_{36}H_{50}N_4O_2S_2.3C_4H_4O_4$:
Berechnet: C, 58,64, H, 6,36, N, 5,70, S, 6,52
Gefunden: C, 58,60, H, 6,51, N, 5,83, S, 6,23.

**Beispiel 16**

Kapseln enthaltend die folgenden Bestandteile können hergestellt werden:

| | Bestandteile | mg/Kapsel | | | | | | | |
|---|---|---|---|---|---|---|---|---|---|
| | | 1 | 5 | 15 | 30 | 60 | 100 | 250 | 500 |
| 1. | (S)-1-(1-Methyläthylamino)-3-[4-[2-[4-[3-(2-chlor-10H-phenothiazin-10-yl)propyl]-piperazin-1-yl)äthoxy]-phenoxy]-2-propanol-trihydrochlorid | 1 | 5 | 15 | 30 | 60 | 100 | 250 | 500 |
| 2. | Milchzucker | 203 | 199 | 239 | 224 | 194 | 99 | 148 | - |
| 3. | Stärke | 30 | 30 | 30 | 30 | 30 | 30 | 30 | 45 |
| 4. | Talk | 15 | 15 | 15 | 15 | 15 | 15 | 15 | 15 |
| 5. | Magnesiumstearat | 1 | 1 | 1 | 1 | 1 | 1 | 2 | 3 |
| | Kapselfüllgewicht | 250 mg | 250 mg | 300 mg | 300 mg | 300 mg | 245 mg | 445 mg | 563 mg |

**Verfahren**

Die Bestandteile 1, 2 und 3 werden in einem geeigneten Mixer vermischt. Der Talk und das Magnesiumstearat werden zugegeben, worauf man nochmals für kurze Zeit gut vermischt. Danach wird in Kapseln geeigneter Grösse abgefült.

**Patentansprüche** für die Vertragsstaaten BE CH DE FR GB IT LI LU NL

SE

1. Verbindungen der allgemeinen Formel

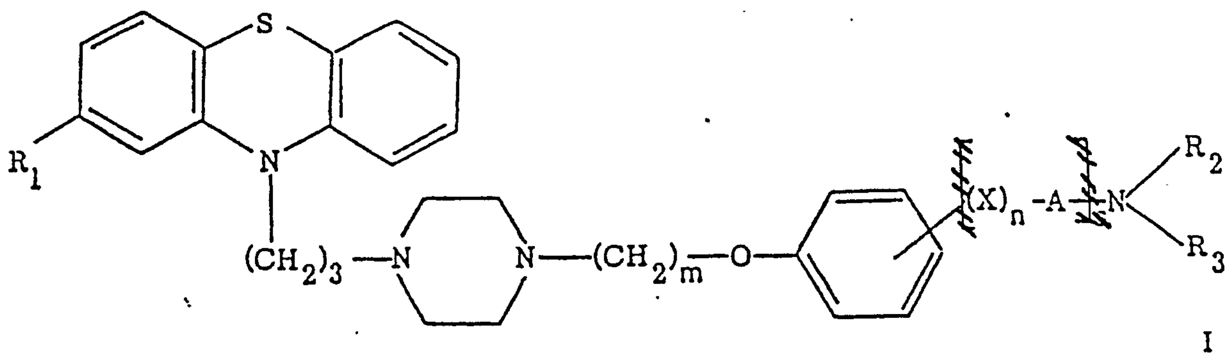

I

worin $R_1$ Wasserstoff, Halogen, $C_1$-$C_7$-Alkyl, $C_1$-$C_7$-Alkyl-thio, $C_1$-$C_7$-Alkoxy, Trihalomethyl oder die Gruppe $-SO_2NR_2R_3$, X Sauerstoff oder Schwefel, m eine Zahl von 2 bis 6, n die Zahl O oder 1, A $C_1$-$C_7$-Alkylen und, falls X Sauerstoff bedeutet, auch 2-Hydroxytrimethylen, und $R_2$ und $R_3$ unabhängig voneinander Wasserstoff oder $C_1$-$C_7$-Alkyl oder zusammen mit dem Stickstoffatom Morpholino oder Phthalimido bedeuten, die Enantiomeren der Verbindungen worin A 2-Hydroxytri-methylen bedeutet und pharmazeutisch verwendbare Säureadditionssalze davon.

2. Verbindungen gemäss Anspruch 1, worin X Sauerstoff und n die Zahl 1 bedeuten.

3. Verbindungen gemäss Anspruch 2, worin $R_2$ und $R_3$ unabhängig voneinander je Wasserstoff oder $C_1$-$C_7$-Alkyl bedeuten.

4. Verbindungen gemäss Anspruch 3 worin $R_1$ Halogen oder Trifluormethyl und m die Zahl 2 bedeuten.

5. (S)-1-(1-Methyläthylamino)-3-[4-[2-[4-[3-(2-chlor-10H-phenothiazin-10-yl)propyl]piperazin-1-yl]äthoxy]-phenoxy]-2-propanol.

6. (R)-1-(1-Methyläthylamino)-3-[4-[2-[4-[3-(2-chlor-10H-phenothiazin-10-yl)propyl]piperazin-1-yl]äthoxy]-phenoxy]-2-propanol, (S)-1-(1-Methyläthylamino)-3-[4-[2-[4-[3-(2-trifluormethyl-10H-phenothiazin-10-yl)propyl]-piperazin-1-yl]äthoxy]phenoxy]-2-propanol und 1-[3-(2-Chlor-10H-phenothiazin-10-yl)propyl]-4-[2-[4-[2-(1-methyl-äthyl)aminoäthoxy]phenoxy]äthyl]piperazin.

7. Eine Verbindung gemäss einem der Ansprüche 1-6 oder ein pharmazeutisch verwendbares Säureadditionssalze davon als pharmazeutischer Wirkstoff.

8. Eine Verbindung gemäss einem der Ansprüche 1-oder ein pharmazeutisch verwendbares Säureadditionssalz davon als Neuroleptikum.

9. Verfahren zur Herstellung von Verbindungen der in Anspruch 1 angegebenen Formel I, der Enantiomeren solcher Verbindungen, worin A 2-Hydroxytrimethylen bedeutet, und von pharmazeutisch verwendbaren Säureadditionssalzen davon, dadurch gekennzeichnet, dass man

a) eine Verbindung der allgemeinen Formel

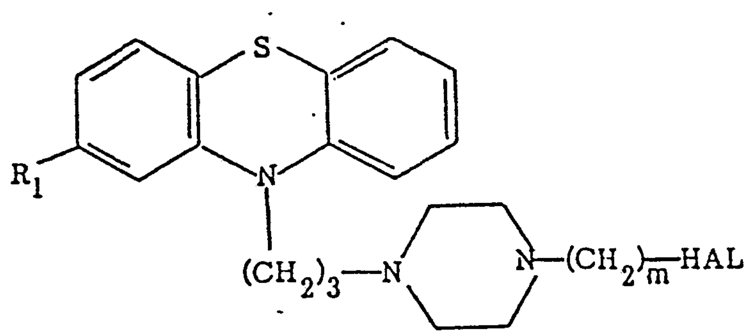

II

worin $R_1$ und m die in Anspruch 1 angegebene Bedeutung besitzen, mit einer Verbindung der allgemeinen Formel

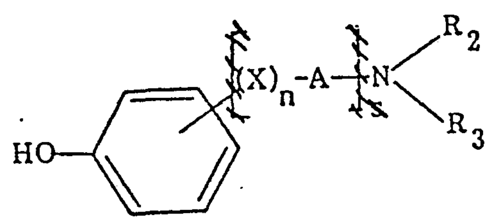

III

worin $R_2$, $R_3$, A, X und n die in Anspruch 1 angegebene Bedeutung besitzen, umsetzt und

b) erwünschtenfalls eine erhaltene Verbindung, worin $R_2$ und $R_3$ zusammen mit dem Stickstoffatom Phthalimido bedeuten, durch Hydrazinolyse in die entsprechende Verbindung überführt, worin $R_2$ und $R_3$ Wasserstoff bedeuten, und/oder

c) erwünschtenfalls ein erhaltenes Racemat einer Verbindung, worin A 2-Hydroxytrimethylen bedeutet, in die optisch aktiven Antipoden auftrennt und

d) erwünschtenfalls eine erhaltene Verbindung oder ein nicht pharmazeutisch verwendbares Säureadditionssalz in ein pharmazeutisch verwendbares Säureadditionssalz überführt.

10. Arzneimittel enthaltend eine Verbindung gemäss einem der Ansprüche 1-6 oder ein pharmazeutisch verwendbared Säureadditionssalze davon.

11. Neuroleptikum enthaltend eine Verbindung gemäss einem der Ansprüche 1-6 oder ein pharmazeutisch verwendbares Säureadditionssalz davon.

**Patentansprüche** den Vertragsstaat AT

1. Verfahren zur Herstellung von Verbindungen der allgemeinen Formel

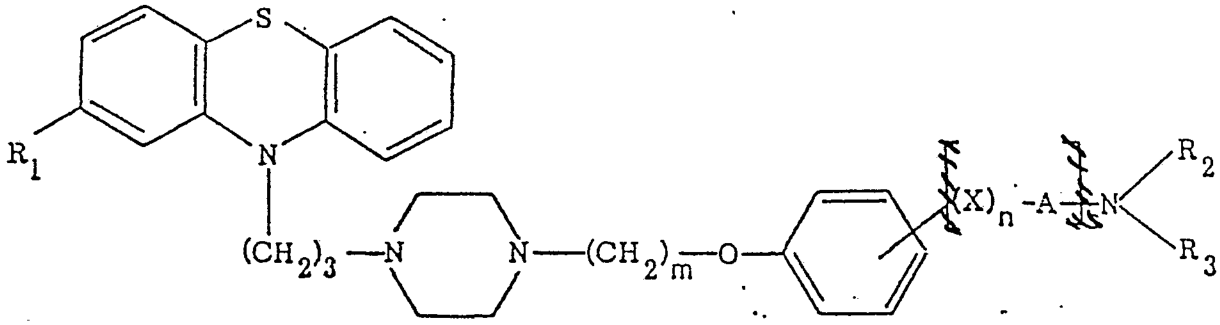

I

worin $R_1$ Wasserstoff, Halogen, $C_1$-$C_7$-Alkyl, $C_1$-$C_7$-Alkylthio, $C_1$-$C_7$-Alkoxy, Trihalomethyl oder die Gruppe -$SO_2NR_2R_3$, X Sauerstoff odar Schwefel, m eine Zahl von 2 bis 6, n die Zahl 0 oder 1, A $C_1$-$C_7$-Alkylan und, falls X Sauerstoff bedeutet, auch 2-Hydroxytrimethylen, und $R_2$ und $R_3$ unabhängig voneinander Wasserstoff oder $C_1$-$C_7$-Alkyl odar zusammen mit dem Stickstoffatom Morpholino odar Phthalimido bedeuten,

der Enantiomeren solcher Verbindungen worin A 2-Hydroxy-trimethylen bedeutet und von pharmazeutisch verwendbaren Säureadditionssalzen davon dadurch gekennzeichnet dass man

a) eine Verbindung der allgemeinen Formel

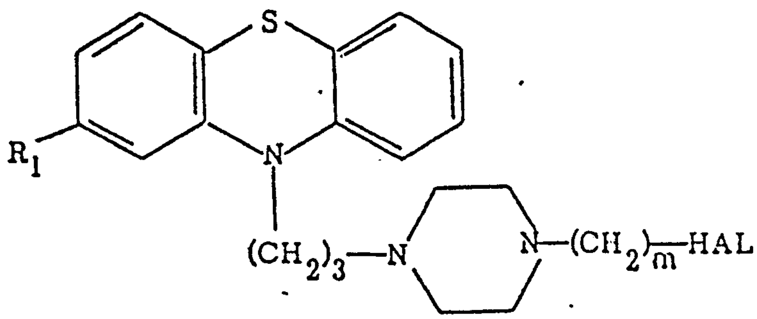

II

worin $R_1$ und m die obige Bedeutung besitzen, mit einer Verbindung der allgemeinen Formel

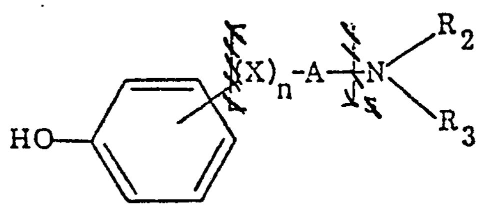

III

worin $R_2$, $R_2$, A, X und n die obige Bedeutung besitzen,
umsetzt und

b) erwünschtenfalls eine erhaltene Verbindung worin $R_2$ und $R_3$ zusammen mit dem Stickstoffatom Phthalimido bedeuten, durch Hydrazinolyse in die entsprechende Verbindung überführt, worin $R_2$ und $R_3$ Wasserstoff bedeuten, und/oder

c) erwünschtenfalls ein erhaltenes Racemat einer Verbindung, worin A 2-Hydroxytrimethylen bedeutet, in die optisch aktiven Antipoden auftrennt und

d) erwünschtenfalls eine erhaltene Verbindung oder ein nicht pharmazeutisch verwendbares Säureadditionssalz in ein pharmazeutisch verwendbares Säureadditionssalz überführt.

2. Verfahren nach Anspruch 1, dadurch gekennzeichnet, dass X Sauerstoff und n die Zahl 1 bedeuten.

3. Verfahren nach Anspruch 2, dadurch gekennzeichnet, dass $R_2$ und $R_3$ unabhängig voneinander Wasserstoff oder $C_1$-$C_7$-Alkyl bedeuten.

4. Verfahren nach Anspruch 3, dadurch gekennzeichnet, dass $R_1$ Halogen oder Trifluormethyl und m die Zahl 2 bedeuten.

5. Verfahren nach Anspruch 1, dadurch gekennzeichnet, dass man (S)-1-(1-Methyläthylamino)-3-[4-[2-[4-[3-(2-chlor-10H-phenothiazin-10-yl)propyl]piperazin-1-yl]äthoxy]-phenoxy]-2-propanol herstellt.

**Claims** for the Contracting Statas: BE CH DE FR GB IT LI LU NL SE

1. Compounds of the general formula

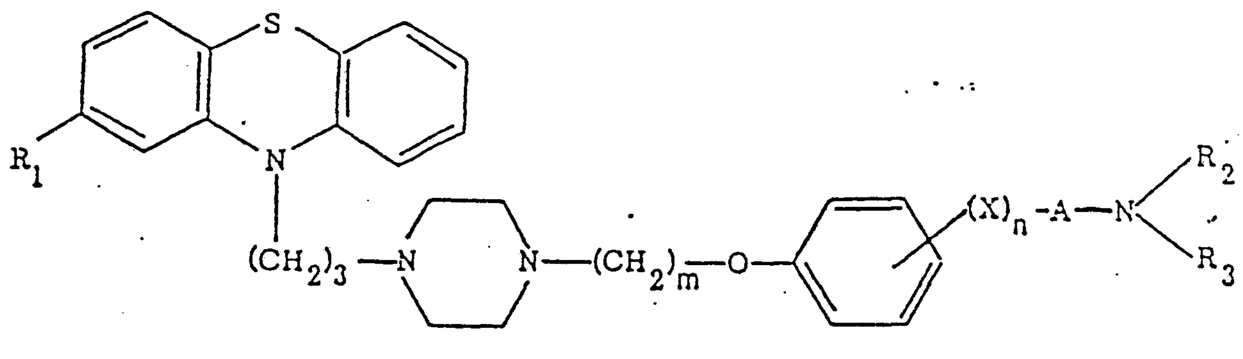

I

wherein $R_1$ signifies hydrogen, halogen, $C_1$-$C_7$-alkyl, $C_1$-$C_7$-alkylthio, $C_1$-$C_7$-alkoxy, trihalomethyl or the group $-SO_2NR_2R_3$, X signifies oxygen or sulphur, m signifies a number from 2 to 6, n signifies the number 0 or 1, A signifias $C_1$-$C_7$-alkylene and, where X signifies oxygan, also 2-hydroxytrimathylane, and $R_2$ and $R_3$ each independantly signify hydrogen or $C_1$-$C_7$-alkyl or together with the nitrogen atom morpholino or phthalimido, the enantiomers of the compounds in which A signifies 2-hydroxytrimetnylene, and pharmaceutically usable acid addition salts thereof.

2. Compounds in accordance with claim 1, wherein X signifies oxygen and n signifies the number 1.

3. Compounds in accordance with claim 2, wherein $R_2$ and $R_3$ each independently signify hydrogen or $C_1$-$C_7$-alkyl.

4. Compounds in accordance witn claim 3, wnerein R signifies halogen or trifluoromethyl and m signifies the number 2.

5. (S)-1-(1-Metnyletnylamino)-3-[4-[2-[4-[3-(2-chloro-10H-phenotniazin-10-yl)propyl]piperazin-1-yl]etnoxy]-pnenoxy]-2-propanol.

6. (R)-1-(1-Metnyletnylamino)-3-[4-[2-[4-[3-(2-chloro-10H-pnenotniazin-10-yl)propyl]piperazin-1-yl]etnoxy]-pnenoxy]-2-propanol, (S)-1-(1-metnyletnylamino)-3-[4-[2-[4-[3-(2-trifluorometnyl-10H-pnenotniazin-10-yl)propyl]-piperazin-1-yl]ethoxy]phenoxy]-2-propanol and 1-[3-(2-chloro-10H-pnenotniazin-10-yl)propyl]-4-[2-[4-[2-(1-methyl-ethyl)aminoetnoxy]phenoxy]ethyl]piperazine.

7. A compound in accordance with any one of claims 1-6 or a pharmaceutically usable acid addition salt tnereof as a pnarmaceutically active substance.

8. A compound in accordance witn any one of claims 1-6 or a pnarmaceutically usable acid addition salt tnereof as a neuroleptic.

9. A process for the manufacture of compounds of formula I given in claim 1, of the enantiomers of those compounds in which A signifies 2-nydroxytrimethylene and of pnarmaceutically usable acid addition salts tnereof, cnaracterized by

a) reacting a compound of the general formula

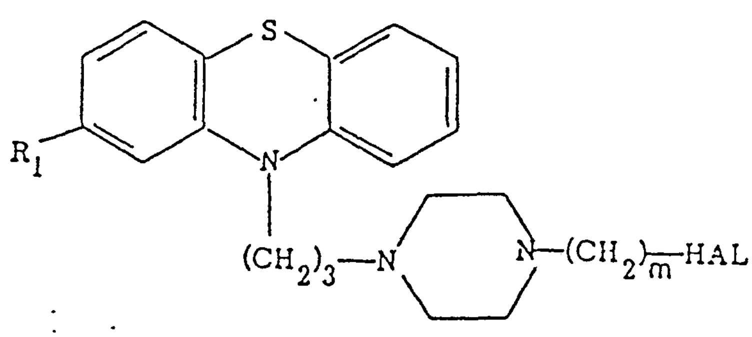

II

where in $R_1$ and m have the significance given in claim 1, with a compound of the general formula

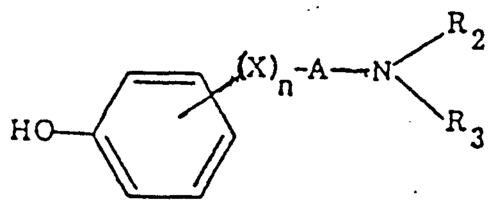

III

wherein $R_2$, $R_3$, A, X and n have the significance given in claim 1, and

b) if desired, converting a compound obtained in which $R_2$ and $R_3$ togetner with the nitrogen atom signify phthalimido into the corresponding compound in which $R_2$ and $R_3$ signify hydrogen by hydrazinolysis, and/or

c) if desired, resolving a racemate of a compound obtained in which A signifies 2-hydroxytrimethylene into the optically active antipodes, and

d) if desired, converting a compound obtained or a non-pharmaceutically usable acid addition salt into a pharmaceutically usable acid addition salt.

10. A medicament containing a compound in accordance with any one of claims 1-6 or a pharmaceutically usable acid addition salt thereof.

11. A neuroleptic containing a compound in accordance with any one of claims 1-6 or a pharmaceutically usable acid addition salt thereof.

**Claims** for the Contracting State: AT

1. A process for the manufacture of compounds of the general formula

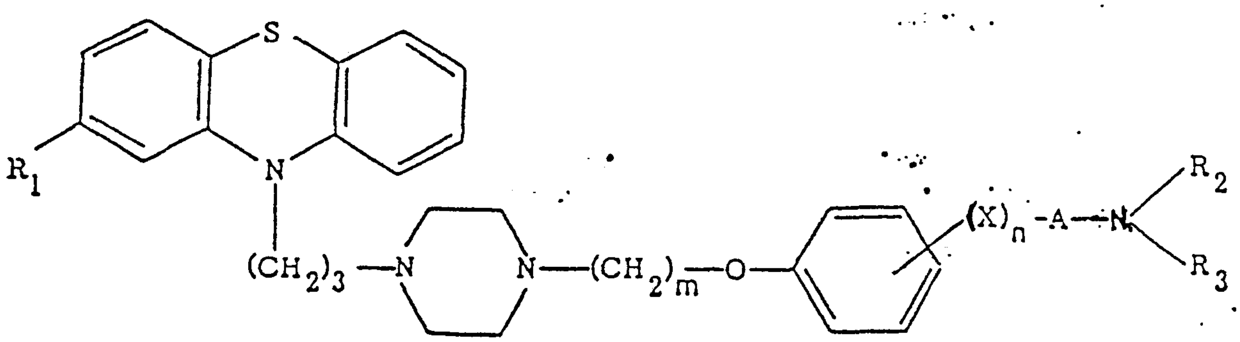

I

wherein $R_1$ signifies hydrogen, halogen, $C_1$-$C_7$-alkyl, $C_1$-$C_7$-alkylthio, $C_1$-$C_7$-alkoxy, trihalomethyl or the group $-SO_2NR_2R_3$, X signifies oxygen or sulphur, m signifies a number from 2 to 6, n signifies the number 0 or 1, A signifies $C_1$-$C_7$-alkylene and, where X signifies oxygen, also 2-hydroxytrimethylene, and $R_2$ and $R_3$ each independently signify hydrogen or $C_1$-$C_7$-alkyl or together with the nitrogen atom morpholino or phthalimido,

of the enantiomers of those compounds in which A signifies 2-hydroxytrimethylene and of pharmaceutically usable acid addition salts thereof, characterized by

a) reacting a compound of the general formula

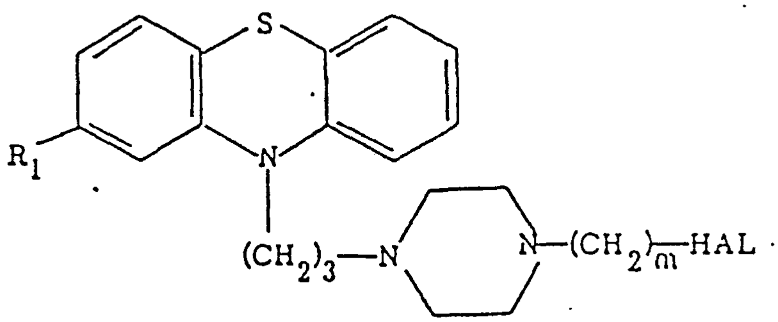

II

wherein $R_1$ and m have the above significance, with a compound of the general formula

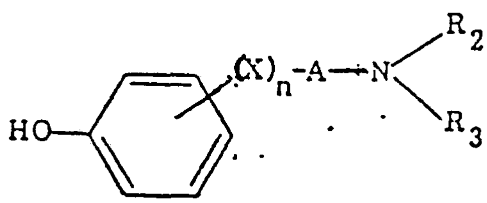

III

wherein $R_2$, $R_3$, A, X and n have the above significance,
and

b) if desired, converting a compound obtained in which $R_2$ and $R_3$ together with the nitrogen atom signify phthalimido into the corresponding compound in which $R_2$ and $R_3$ signify hydrogen by hydrazinolysis, and/or

c) if desired, resolving a racemate of a compound obtained in which A signifies 2-hydroxytrimethylene into the optically active antipodes, and

d) if desired, converting a compound obtained or a non-pharmaceutically usable acid addition salt into a pharmaceutically usable acid addition salt.

2. A process according to claim 1, characterized in that X signifies oxygen and n signifies the number 1.

3. A process according to claim 2, characterized in that $R_2$ and $R_3$ each independently signify hydrogen or $C_1$-$C_7$-alkyl.

4. A process according to claim 3, characterized in that $R_1$ signifies halogen or trifluoromethyl and m signifies the number 2.

5. A process according to claim 1, characterized in that (S)-1-(1-methylethylamino)-3-[4-[2-[4-[3-(2-chloro-10H-phenothiazin-10-yl)propyl]piperazin-1-yl]ethoxy]-phenoxy]-2-propanol is manufactured.

**Revendications** pour les Etats contactants: BE, CH, DE, FR, GB, IT, LI,

LU, NL, SE

1 - Composés de formule générale

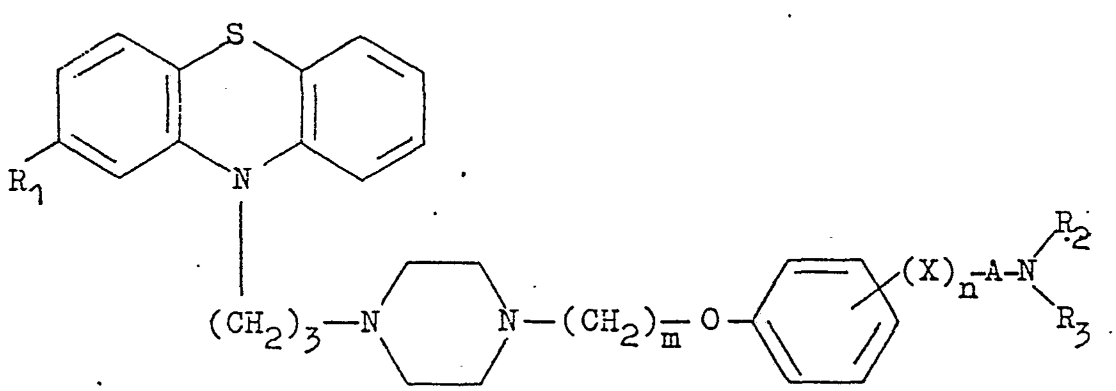

I

dans laquelle $R_1$ représente l'hydrogéne, un halogène, un groupe alkyle en C1-C7, alkylthio en C1-C7, alcoxy en C1-C7,trihalogénométhyle ou $-SO_2NR_2R_3$, X représente l'oxygène ou le soufre, m est un nonbre allant de 2 à 6, n est égal à 0 ou 1, A représente un groupe alkylène en C1-C7 ou encore, lorsque X représente l'oxygène, un groupe 2-hydroxytrimiéthylène, et $R_2$et $R_3$ représentent chacun, indépendamment l'un de l'autre, l'hydrogène ou un groupe alkyle en C1-C7, ou bien, ensemble et avec l'atome d'azote, un groupe morpholino ou phthalimido, les énantiomères des composés dans lesquels A représente un groupe 2-hydroxytriméthylene, et leurs sels formés par addition avec des acides acceptables pour l'usage pharmaceutique.

2 - Composés selon la revendication 1, dans lesquels X représente l'oxygène et n est égal à 1.

3 - Composés selon la revendication 2, dans lesquels $R_2$ et $R_3$ représentent chacun, indépendamment l'un de l'autre, l'hydrogène ou un groupe alkyle en C1-C7.

4 - Composés selon la revendication 3, dans lesquels $R_1$ représente un halogène ou un groupe trifluorométhyle et m est égal à 2.

5 - Le (S)-1-(1-méthyléthylamino)-3[4-[2-[4-[3-(2-chloro-10H-phénothiazine-10-yl)-propyl]-pipérazine-1-yl]-éthoxy]-phénoxy]-2-propanol.

6 - Le (R)-1-(1-méthyléthylamino)-3-[4-[2-4[-[3-(2-chloro-10H-phénothiazine-10-yl)-propyl]-pipérazine-1-yl]-éthoxy]-phénoxy]-2-propanol, le (S)-1-(1-méthyl-ethylamino)-3-[4-[2-[4-[3-(2-trifluorométhyl-10H-phénothiazine-10-yl)-propyl)-pipérazine-1-yl]-éthoxy]-phénoxy]-2-propanol et la 1-[3-(2-chloro-10H-phénothiazine-10-yl)-propyl]-4-[2-[4-[2-(1-méthyléthyl)-aminoéthoxy]-phénoxy]-éthyl]-pipérazine.

7 - Un composé selon l'une des revendications 1 à 6 ou un sel d'un tel composé formé par addition avec un acide acceptable pour l'usage pharmaceutique, en tant que substance active pharmaceutique.

8 - Un composé selon l'une des revendications 1 à 6 ou un sel d'un tel composé formé par addition avec un acide acceptable pour l'usage pharmaceutique, en tant que neuroleptique.

9 - Procédé de préparation des composés de formule I de la revendication 1, des énantiomères des composés dans lesquels A représente un groupe 2-hydroxytriméthylène et de leurs sels formés par addition avec des acides acceptables pour l'usage pharmaceutique, caractérisé en ce que:

a) on fait réagir un compoaé de formule générale

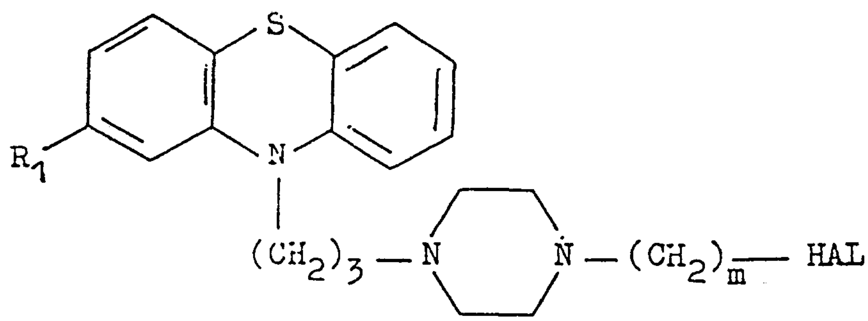

II

dans laquelle $R_1$ et m ont les significations indiquées dans la revendication 1, avec un composé de formule générale

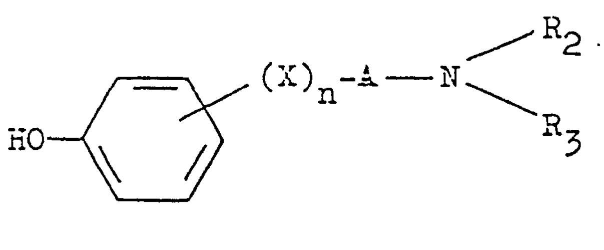

III

dans laquelle $R_2$, $R_3$, A, X et n ont les significations indiquées dans la revendication 1, et

b) si on le désire, on convertit un composé obtenu dans lequel $R_2$ et $R_3$ forment ensemble et avec l'atome d'azote le kroupe phthalimido, par hydrazinolyse, en le compose correspondant dans lequel $R_2$ et $R_3$ représentent l'hydrogéne, et/ou

c) si on le désire, on sépare un racémate d'un composé dans lequel A représente le groupe 2-hydroxytriméthylène, ainsi obtenu, en aes antipodes optiques, et

d) si on le désire, on convertit un composé obtenu ou un sel formé par addition avec un acide non acceptable pour l'usage pharmaceutique en un sel formé par addition avec un acide acceptable pour l'usage pharmaceutique.

10 - Médicament contenant un composé selon l'une des revendications 1 à 6 ou un sel d'un tel composé formé par addition avec un acide acceptable pour l'usage pharmaceutique.

11 - Neuroleptique contenant un composé selon l'une des revendications 1 à 6 ou un sel d'un tel composé formé par addition avec un acide acceptable pour l'usage pharmaceutique.

**Revendications** pour l'Etat contractant: AT

1 - Procédé de préparation des composés de formule générale

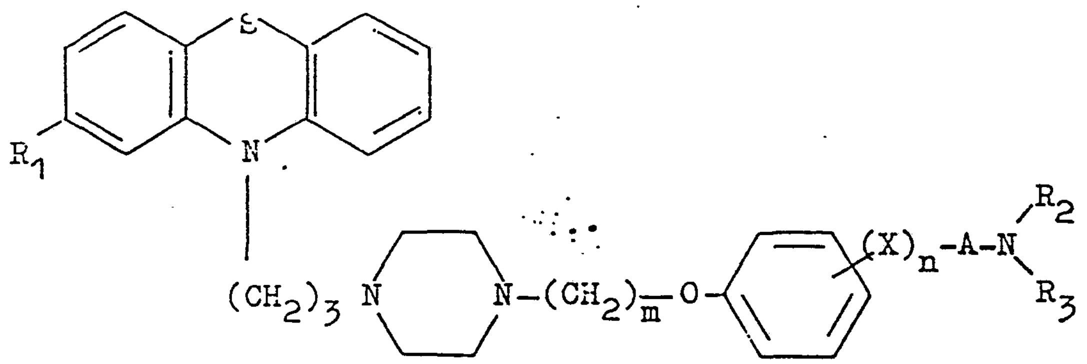

I

dans laquelle $R_1$ représente l'hydrogène, un halogène, un groupe alkyle en C1-C7, alkylthio en C1-C7, alcoxy en C1-C7, trihalogénométhyle ou le groupe $-SO_2NR_2R_3$, X représente l'oxygène ou le soufre, m est un nombre allant de 2 à 6, n est égal à 0 ou 1, A représente un groupe alkylène en C1-C7 ou bien encore, lorsque X représente l'oxygène, un groupe 2-hydroxytriméthylène, et $R_2$ et $R_3$ représentent, indépendamment l'un de l'autre, l'hydrogène ou un groupe alkyle en C1-C7 ou bien, ensemble et avec l'atome d'azote, un groupe morpholino ou phthalimido, des énantiomères de ces composés dans lesquels A représente un groupe 2-hydroxytriméthylène, et de leurs sels formés par addition avec des acides acceptables pour l'usage pharmaceutique, caracterise en ce que:

a) on fait réagir un composé de formule générale

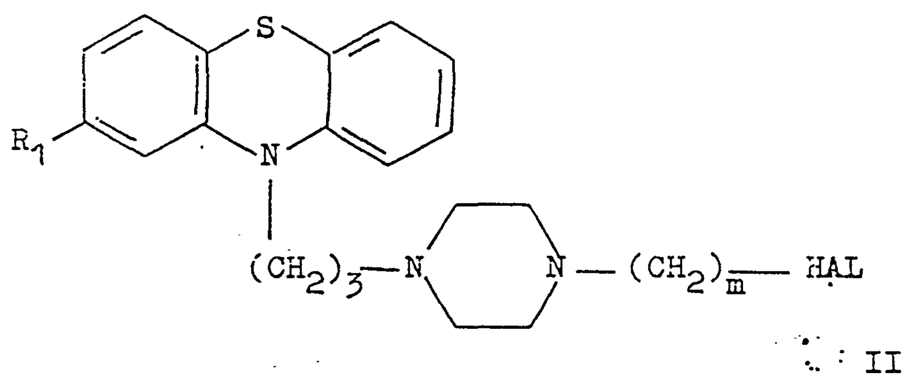

II

dans laquelle $R_1$ et m ont les significations indiquées ci-dessus,
avec un composé de formule générale

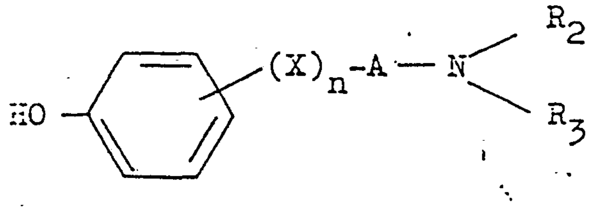

III

dans laquelle $R_2$, $R_3$, A, X et n ont les significations indiquées ci-dessus, et

b) si on le désire, on convertit un composé obtenu dans lequel $R_2$ et $R_3$ forment ensemble et avec l'atome d'azote un groupe phtalimido, par hydrazinolyse, en le composé correspondant dans lequel $R_2$ et $R_3$ représentent l'hydrogéne, et/ou

c) si on le désire, on sépare un racémate d'un composé dans lequel A représente le kroupe 2-hydroxytriméthylène, ainsi obtenu, en ses antipodes optiques, et

d) si on le désire, on convertit un composé obtenu ou un sel formé par addition avec un acide non acceptable pour l'usage pharmaceutique en un sel formé par addition avec un acide acceptable pour l'usage pharmaceutique.

2 - Procédé selon la revendication 1, caractérisé en ce que X représente l'oxygéne et n est égal à 1.

3 - Procédé selon la revendication 2, caractérisé en ce que $R_2$ et $R_3$ représentent chacun, indépendamment l'un de l'autre, l'hydrogène ou un groupe alkyle en Cl-C7.

4 - Procédé selon la revendication 3, caractérisé en ce que $R_1$ représente un halogène ou un groupe trifluorométhyle et m est égal à 2.

5 - Procédé selon la revendication 1, caractérisé en ce que l'on prépare le (S)-1-(1-méthyléthylamino)-3-[4-[2-[4-[3-(2-chloro-10H-phénothiazine-10-yl)-propyl)]-pipérazine-1-yl[-éthoxy]-phénoxy]-2-propanol.